# EUROPEAN PATENT APPLICATION

(11) **EP 3 895 688 A1**
(43) Date of publication of application: **20.10.2021**
(21) Application number: 19894482.9
(22) Date of filing: 06.12.2019
(51) Int. Cl.: A61K 8/81, A61K 8/04, A61K 8/31, A61K 8/34, A61K 8/37, A61Q 5/02, A61Q 19/10, C11D 3/18, C11D 3/20, C11D 3/30, C11D 3/32, C11D 3/37, C11D 7/24, C11D 7/26, C11D 7/32, D06F 35/00

(54) **SALT-SENSITIVE PARTICLES**

(30) Priority: 12.12.2018 JP 2018232839; 21.10.2019 JP 2019192169
(71) Applicant: Kao Corporation, Chuo-ku, Tokyo 103-8210 (JP)
(72) Inventor: NONAKA, Nobuhiro, Wakayama-shi, Wakayama 640-8580 (JP); MAETA, Yuya, Wakayama-shi, Wakayama 640-8580 (JP); WARITA, Hiroaki, Wakayama-shi, Wakayama 640-8580 (JP); ZHANG, Jicheng, Tokyo 131-8501 (JP); TAKATO, Kenji, Wakayama-shi, Wakayama 640-8580 (JP); OZAWA, Toshiaki, Tokyo 131-8501 (JP)
(74) Representative: Hoffmann Eitle
(86) International application number: PCT/JP2019/047771
(87) International publication number: WO 2020/121958

(57) **Abstract**

The present invention relates to the provision of salt-sensitive particles capable of improving the feeling of effects at the time of use of an oil agent to be blended in a cleaner or the like and a method for producing the same, and so on.

The present invention provides the following [1] to [4].
[1] Salt-sensitive particles containing an oil agent having a solubility in 100 g of water of less than 1 g and an acid-modified polyvinyl alcohol, wherein the oil agent is dispersed in the salt-sensitive particles.
[2] A cleaner containing the salt-sensitive particles as set forth in [1].
[3] A method for cleaning a skin, a hair, or a clothing, including using the cleaner as set forth in [2].
[4] A method for producing the salt-sensitive particles as set forth in [1], including a step of preparing an emulsion composition containing an oil agent having a solubility in 100 g of water of less than 1 g and an acid-modified polyvinyl alcohol; and a step of removing the water from the emulsion composition.

## Description

### Field of the Invention

The present invention relates to salt-sensitive particles, a cleaner containing the salt-sensitive particles, a cleaning method, and a method for producing the salt-sensitive particles.

### Background of the Invention

There are known cleaning agents having polymer particles blended therein for various purposes.

For example, JP 6-219924 A (PTL 1) discloses a facial cleaner containing microcapsules including one or more kinds of oil agents therein, in which polyvinyl alcohol having a particle diameter of 10 to 1,000 pm and having a high crystallinity is provided as a film material, for the purpose of solving a problem that during the production or storage of the facial cleaner, the contents of the capsule in a surfactant of the facial cleaner do not bleed out, the capsule is free from precipitation and can be easily disintegrated by a pressure of hand when using, and sufficient functions may be thoroughly exhibited.

In addition, JP 2014-108952 (PTL 2) discloses pigment granules containing a pigment, polyvinylpyrrolidone, and polyvinyl alcohol, wherein a viscosity characteristic value (K value) of the polyvinylpyrrolidone is 25 or less, for the purpose of providing pigment granules capable of suppressing coloration at the time of storage of a cleaning agent and allowing foams to quickly develop a color at the time of cleaning.

### Summary of the Invention

The present invention relates to salt-sensitive particles containing an oil agent having a solubility in 100 g of water of less than 1 g and an acid-modified polyvinyl alcohol, wherein the oil agent is dispersed in the salt-sensitive particles.

### Advantageous Effects of the Invention

In accordance with the present invention, salt-sensitive particles capable of improving the feeling of effects at the time of use of an oil agent to be blended in a cleaner or the like and a method for producing the same, and so on can be provided. Furthermore, in accordance with the present invention, a cleaner containing the salt-sensitive particles and a cleaning method of using the cleaner can be provided.

### Detailed Description of the Invention

The facial cleaner in PTL 1 contains the oil agent in the microcapsule and is excellent in stability during the storage and easy disintegrability at the time of use. However, the facial cleaner in PTL 1 is produced by the coacervation method, and the oil agent is not dispersed in the capsule, and thus, it may be not said that the effect at the time of use is sufficient.

In addition, in PTL 2, polyvinyl alcohol is used as one of salt-sensitive binders, and it is described that coloration is suppressed at the time of storage of the cleaning agent, and the foams are allowed to quickly develop a color at the time of cleaning. However, in PTL 2, the oil agent is not dispersed and contained.

The present invention is to provide salt-sensitive particles capable of improving the feeling of effects at the time of use of an oil agent to be blended in a cleaner or the like and a method for producing the same, and so on. Furthermore, the present invention is to provide a cleaner containing the salt-sensitive particles and a cleaning method of using the cleaner.

In view of the aforementioned problem, the present inventors made extensive and intensive investigations. As a result, it has been found that salt-sensitive particles in which an oil agent is dispersed in a polymer containing an acid-modified polyvinyl alcohol improve the feeling of effects on the basis of the oil agent at the time of use. The wording "at the time of use" means at least one of "during the use" and "after the use". The same is also applicable to the wording "at the time of cleaning".

Specifically, the present invention provides the following [1] to [4].
[1] Salt-sensitive particles containing an oil agent having a solubility in 100 g of water of less than 1 g and an acid-modified polyvinyl alcohol, wherein the oil agent is dispersed in the salt-sensitive particles.
[2] A cleaner containing the salt-sensitive particles as set forth in [1].
[3] A method for cleaning a skin, a hair, or a clothing, including using the cleaner as set forth in [2].
[4] A method for producing the salt-sensitive particles as set forth in [1], including a step of preparing an emulsion composition containing an oil agent having a solubility in 100 g of water of less than 1 g and an acid-modified polyvinyl alcohol; and a step of removing the water from the emulsion composition.

In accordance with the present invention, salt-sensitive particles capable of improving the feeling of effects at the time of use of an oil agent to be blended in a cleaner or the like and a method for producing the same, and so on can be provided. Furthermore, in accordance with the present invention, a cleaner containing the salt-sensitive particles and a cleaning method of using the cleaner can be provided.

### [Salt-Sensitive Particles]

The salt-sensitive particles of the present invention are salt-sensitive particles containing an oil agent having a solubility in 100 g of water of less than 1 g and an acid-modified polyvinyl alcohol, wherein the oil agent is dispersed in the salt-sensitive particles.

In the present invention, the "salt-sensitive particles" mean particles whose solubility varies with a concentration of a water-soluble salt (for example, sodium chloride) in the composition containing the salt-sensitive particles. In the present invention, the salt-sensitive particles are particles whose solubility is improved when the concentration of the water-soluble salt in the composition containing the salt-sensitive particles is decreased.

As for the reason why the feeling of effects of an oil agent of a cosmetic, such as a cleaner using the salt-sensitive particles of the present invention, is improved, it may be considered that in the salt-sensitive particles having the oil agent dispersed therein, in view of the fact that the salt concentration is decreased at the time of diluting the cleaner (at the time cleaning and at the time of rinsing), the particles are easily disintegrated, and not only the oil agent in the particles is easily released, but also the particle strength is lowered, and a physical force is applied by a finger or the like, and the oil agent is much more easily released, and therefore, the feeling of effects of the oil agent is improved.

In the present invention, the oil agent is dispersed in the salt-sensitive particles, and it may be considered that in comparison of the same amount of the oil agent, by using particles containing a more finely dispersed oil agent, permeability of the oil agent into the skin is improved, and the feeling of effects is improved. In addition, since the oil agent is contained in the salt-sensitive particles, a cosmetic, such as a cleaner containing the foregoing particles is able to suppress the oil agent from separation, and is excellent in storage stability.

Furthermore, on the occasion of washing a face with the cleaning agent composition containing the salt-sensitive particles of the present invention, coupled with a water brought from hands and fingers and a physical force by fingers, etc., the disintegrability of the salt-sensitive particles on a cheek and forehead of the face is improved, and therefore, it may be considered that the effects of the oil agent on the cheek and forehead (for example, a feeling of coolness of menthol) is strongly felt. On the other hand, the disintegration of the salt-sensitive particles is suppressed in eyes, and therefore, it may be considered that a burning sensation in the eyes can be suppressed.

As for the salt-sensitive particles of the present invention, a plurality of pores (derived from the oil agent) (2 or more) preferably exist in the cross section obtained by cleaving the particles by the method as mentioned later, and the pores exist in the number of more preferably 5 or more, and still more preferably 10 or more. Specifically, the number of pores is preferably 2 or more, more preferably 5 or more, and still more preferably 10 or more per 0.001 mm² of the cross-sectional area of the particles.

### (Acid-Modified Polyvinyl Alcohol)

The acid-modified polyvinyl alcohol to be used in the present invention is a polyvinyl alcohol having an acid group, such as a sulfonic acid group, a sulfuric acid group, a carboxylic acid group, a phosphoric acid group, and a phosphonic acid group. From the viewpoint of storage stability in a product, such as a cleaner, and disintegrability owing to a decrease of the concentration of the water-soluble salt at the time of cleaning (during cleaning and at the time of rinsing), an acid-modified polyvinyl alcohol having at least one of a sulfonic acid group and a carboxylic acid group introduced thereinto is preferred, and an acid-modified polyvinyl alcohol having a carboxylic acid group introduced thereinto (hereinafter also referred to as "carboxylic acid-modified polyvinyl alcohol") is more preferred.

Examples of the carboxylic acid-modified polyvinyl alcohol include (1) one obtained by graft polymerizing or block polymerizing polyvinyl alcohol and an unsaturated monomer having a carboxy group; (2) one obtained by copolymerizing a vinyl ester compound and an unsaturated monomer having at least one selected from a carboxy group (carboxylic acid group) and a carboxylic acid ester group, followed by saponification; (3) one obtained by polymerizing a vinyl ester compound using a chain transfer agent having a carboxy group, followed by saponification; and (4) one obtained by reacting polyvinyl alcohol with a carboxylating agent.

Examples of the unsaturated monomer having a carboxy group to be used in the aforementioned methods (1) and (2) and the unsaturated monomer having a carboxylic acid ester group to be used in the method (2) include ethylenically unsaturated dicarboxylic acids, such as maleic acid, fumaric acid, and itaconic acid; ethylenically unsaturated dicarboxylic acid monoesters, such as a maleic acid monoalkyl ester, a fumaric acid monoalkyl ester, and an itaconic acid monoalkyl ester; ethylenically unsaturated dicarboxylic acid diesters, such as a maleic acid dialkyl ester, a fumaric acid dialkyl ester, and an itaconic acid dialkyl ester; ethylenically unsaturated carboxylic acid anhydrides, such as maleic anhydride and itaconic anhydride; unsaturated monocarboxylic acids, such as (meth)acrylic acid; and unsaturated monocarboxylic acid esters, such as a (meth)acrylic acid alkyl ester. In addition, salts of the aforementioned compounds may also be used as the unsaturated monomer having at least one selected from a carboxy group and a carboxylic acid ester group.

Of these, from the viewpoint of reactivity, ethylenically unsaturated carboxylic acid monoesters are preferred; ethylenically unsaturated dicarboxylic acid monoesters are more preferred; a maleic acid monoalkyl ester and an itaconic acid monoalkyl ester are still more preferred; and a maleic acid monoalkyl ester is yet still more preferred.

These compounds may be used alone or may be used in combination of two or more thereof.

Examples of the vinyl ester compound to be used in the aforementioned methods (2) and (3) include vinyl acetate, vinyl formate, vinyl propionate, vinyl versatate, and vinyl pivalate. Of these, vinyl acetate is preferred from the viewpoint of reactivity at the time of synthesis and easiness of availability.

These compounds may be used alone or may be used in combination of two or more thereof.

Examples of the carboxylating agent to be used in the aforementioned method (4) include carboxylic acid anhydrides, such as succinic anhydride, maleic anhydride, acetic anhydride, trimellitic anhydride, phthalic anhydride, pyromellitic anhydride, glutaric anhydride, hydrogenated phthalic anhydride, and naphthalene dicarboxylic anhydride.

These may be used alone or may be used in combination of two or more thereof.

An acid modification rate in the acid-modified polyvinyl alcohol (ratio of the monomer having an acid group) is preferably 0.1 mol% or more, more preferably 0.5 mol% or more, and still more preferably 1 mol% or more from the viewpoint of improving releasability of the oil agent owing to a decrease of the concentration of the water-soluble salt, and it is preferably 10 mol% or less, more preferably 5 mol% or less, and still more preferably 3 mol% or less from the viewpoint of storage stability of the salt-sensitive particles in the product. In consequence, the acid modification rate in the acid-modified polyvinyl alcohol is preferably 0.1 mol% or more and 10 mol% or less, more preferably 0.5 mol% or more and 5 mol% or less, and still more preferably 1 mol% or more and 3 mol% or less from the viewpoint of improving releasability of the oil agent owing to a decrease of the concentration of the water-soluble salt and the viewpoint of storage stability of the salt-sensitive particles in the product.

The acid modification rate in the acid-modified polyvinyl alcohol can be determined by analyzing the acid-modified polyvinyl alcohol before saponification with ¹H-NMR (solvent: CDCl₃).

A degree of saponification of the acid-modified polyvinyl alcohol is 70 mol% or more, more preferably 80 mol% or more, and still more preferably 90 mol% or more from the viewpoint of storage stability of the salt-sensitive particles in the product, and it is preferably 99.9 mol% or less, more preferably 99.5 mol% or less, and still more preferably 99 mol% or less from the viewpoint of improving releasability of the oil agent owing to a decrease of the concentration of the water-soluble salt. In consequence, the degree of saponification of the acid-modified polyvinyl alcohol is preferably 70 mol% or more and 99.9 mol% or less, more preferably 80 mol% or more and 99.5 mol% or less, and still more preferably 90 mol% or more and 99 mol% or less from the viewpoint of improving releasability of the oil agent owing to a decrease of the concentration of the water-soluble salt and the viewpoint of storage stability of the salt-sensitive particles in the product.

The degree of saponification of the acid-modified polyvinyl alcohol is measured in conformity with JIS K6726:1994.

A degree of polymerization of the acid-modified polyvinyl alcohol is preferably 100 or more, more preferably 500 or more, and still more preferably 1,000 or more from the viewpoint of storage stability of the salt-sensitive particles (granules) in the product, and it is preferably 200,000 or less, more preferably 10,000 or less, and 4,000 or less from the viewpoint of improving releasability of the oil agent owing to a decrease of the concentration of the water-soluble salt. In consequence, the degree of polymerization of the acid-modified polyvinyl alcohol is preferably 100 or more and 200,000 or less, more preferably 500 or more and 10,000 or less, and still more preferably 1,000 or more and 4,000 or less from the viewpoint of improving releasability of the oil agent owing to a decrease of the concentration of the water-soluble salt and the viewpoint of storage stability of the salt-sensitive particles in the product.

The degree of polymerization of the acid-modified polyvinyl alcohol can be calculated from a relative viscosity between a completely saponified polyvinyl alcohol aqueous solution and water (see JIS K6726:1994).

A molecular weight of the acid-modified polyvinyl alcohol is preferably 5,000 or more, more preferably 10,000 or more, still more preferably 30,000 or more, and yet still more preferably 50,000 or more from the viewpoint of storage stability of the salt-sensitive particles in the product, and it is preferably 1,000,000 or less, more preferably 500,000 or less, and still more preferably 200,000 from the viewpoint of improving releasability of the oil agent owing to a decrease of the concentration of the water-soluble salt. In consequence, the molecular weight of the acid-modified polyvinyl alcohol is preferably 5,000 or more and 1,000,000 or less, more preferably 10,000 or more and 500,000 or less, still more preferably 30,000 or more and 200,000 or less, and yet still more preferably 50,000 or more and 200,000 or less from the viewpoint of improving releasability of the oil agent owing to a decrease of the concentration of the water-soluble salt and the viewpoint of storage stability of the salt-sensitive particles in the product.

The molecular weight of the acid-modified polyvinyl alcohol can be determined through calculation from the degree of polymerization.

Specific examples of the acid-modified polyvinyl alcohol include KL-118, KL-318, KL-506, KM-118, and KM-618, all of which are manufactured by Kuraray Co., Ltd.; GOHSENX CKS50, GOHSENX T-330H, GOHSENX T-330, and GOHSENX T-350, all of which are manufactured by The Nippon Synthetic Chemical Industry Co., Ltd.; and AP-17, AT-17, and AF-17, all of which are manufactured by JAPAN VAM & POVAL CO., LTD.

### (Oil Agent)

The salt-sensitive particles of the present invention contain an oil agent. The oil agent is an organic compound having a solubility in 100 g of water of less than 1 g.

The solubility of the oil agent in 100 g of water is a solubility at 25°C (1,013.25 hPa). From the viewpoint of dispersibility in the salt-sensitive particles, the solubility of the oil agent in 100 g of water is less than 1 g, preferably 0.5 g or less, more preferably 0.3 g or less, and still more preferably 0.1 g or less, and it may also be 0 g; and it is preferably 0 g or more and less than 1 g, more preferably 0 g or more and 0.5 g or less, still more preferably 0 g or more and 0.3 g or less, and yet still more preferably 0 g or more and 0.1 g or less. The measurement of the solubility can be made by reference to, for example, "Journal of the Chemical Society of Japan", 1985, No. 11, pp.2116-2119; and "Journal of the Chemical Society of Japan", 1982, No. 11, pp.1830-1834.

From the viewpoint of preparing an emulsion composition, a melting point of the oil agent is preferably lower than the melting point of water, preferably lower than 100°C, more preferably 99°C or lower, still more preferably 95°C or lower, yet still more preferably 90°C or lower, even yet still more preferably 80°C or lower, even still more preferably 70°C or lower, and even still more further preferably 60°C or lower. In addition, the melting point of the oil agent is preferably -100°C or higher, more preferably 0°C or higher, still more preferably 10°C or higher, and yet still more preferably 20°C or higher from the viewpoint of containing the oil agent in the particles. In consequence, the melting point of the oil agent is preferably -100°C or higher and lower than 100°C, more preferably 0°C or higher and 99°C or lower, still more preferably 0°C or higher and 95°C or lower, yet still more preferably 10°C or higher and 90°C or lower, even yet still more preferably 10°C or higher and 80°C or lower, even still more preferably 20°C or higher and 70°C or lower, and even still more further preferably 20°C or higher and 60°C or lower from the viewpoint of preparing the emulsion composition and the viewpoint of containing the oil agent in the particles.

A molecular weight of the oil agent is preferably 10,000 or less, more preferably 6,000 or less, still more preferably 1,000 or less, and yet still more preferably 500 or less from the viewpoint of dispersing the oil agent in the particles, and it is preferably 80 or more, and still more preferably 100 or more from the viewpoint of stably holding in the particles. In consequence, the molecular weight of the oil agent is preferably 80 or more and 10,000 or less, more preferably 100 or more and 6,000 or less, still more preferably 100 or more and 1,000 or less, and yet still more preferably 100 or more and 500 or less from the aforementioned viewpoint.

In the case where the molecular weight of the oil agent has a distribution, the aforementioned molecular weight means a weight average molecular weight and is a value obtained through measurement by means of gel permeation chromatography and expression by using monodispersed polystyrene having an already-known molecular weight as a standard substance.

Examples of the oil agent include a liquid oil that is a liquid at 20°C and a solid fat that is a solid at 20°C. As the oil agent, a liquid oil alone may be contained, a solid fat alone may be contained, and both of them may be contained. In the case of using a solid fat, an oil ingredient is heated at a temperature of a melting point of the solid fat and melted. The oil agent preferably contains a solid fat from the viewpoint of stably holding in the particles.

Examples of the oil agent include alcohols, ester oils, hydrocarbon oils, silicone oils, dialkyl ether compounds, amine compounds, amide compounds, oils and fats, and higher fatty acids. The oil agent is preferably at least one selected from alcohols, ester oils, hydrocarbon oils, silicone oils, dialkyl ether compounds, amine compounds, amide compounds, oils and fats, and higher fatty acids.

Examples of the alcohol include higher alcohols, alicyclic alcohols, and aromatic alcohols.

Examples of the higher alcohol include saturated or unsaturated, linear or branched alcohols. The higher alcohol is preferably a saturated or unsaturated alcohol, and preferably a branched alcohol. The carbon number of the higher alcohol is preferably 8 or more, more preferably 10 or more, still more preferably 12 or more, yet still more preferably 16 or more, and even yet still more preferably 18 or more from the viewpoint of stably holding in the particles, and it is preferably 22 or less from the same viewpoint. Specifically, examples of the liquid oil include 2-octyldodecan-1-ol; and examples of the solid fat include myristyl alcohol, cetyl alcohol, cetostearyl alcohol, stearyl alcohol, arachidyl alcohol, and behenyl alcohol. These may also be a moisturizing ingredient.

Examples of the alicyclic alcohol include fragrances, such as menthol and cedrol, and refreshing agents.

Examples of the aromatic alcohol include disinfectants, such as isopropyl methylphenol and triclosan.

Examples of the ester oil include a neopentyl glycol difatty acid ester, an ethylene glycol difatty acid ester, and a fatty acid glyceride, such as a fatty acid monoglyceride, a fatty acid diglyceride, and a fatty acid triglyceride. These may also be used as a moisturizing ingredient.

The carbon number of the acyl group of the ester oil is preferably 6 or more, and more preferably 8 or more from the viewpoint of stably holding in the particles, and it is preferably 22 or less, more preferably 18 or less, still more preferably 16 or less, yet still more preferably 14 or less, and even yet still more preferably 12 or less from the same viewpoint.

In addition, examples of the ester oil include dipentaerythrityl pentaisostearate, dipentaerythrityl tetraisostearate, and dipentaerythrityl tripolyhydroxystearate. Examples of a commercially available product of the dipentaerythrityl pentaisostearate include "SALACOS DP-518N", and examples of a commercially available product of the dipentaerythrityl tripolyhydroxystearate include "SALACOS WO-6" (all of which are manufactured by The Nisshin OilliO Group, Ltd.). These can be used alone or in combination of two or more thereof, as the need arises.

Furthermore, there are exemplified organic ultraviolet absorbers, such as 2-ethylhexyl paramethoxycinnamate.

Examples of the hydrocarbon oil include a paraffin, squalene, and squalane. The hydrocarbon oil may be a linear or branched hydrocarbon, may be a saturated or unsaturated hydrocarbon, and may be a cyclic hydrocarbon. The carbon number of the hydrocarbon is preferably 10 or more, more preferably 16 or more, still more preferably 22 or more, and yet still more preferably 28 or more from the viewpoint of stably holding in the particles, and it is preferably 50 or less, more preferably 40 or less, and still more preferably 32 or less from the same viewpoint.

Examples of the paraffin include paraffin waxes and microcrystalline waxes described in JIS K2235:2009, ceresin, soft waxes, vaseline, and paraffins of Japanese Pharmacopoeia, JP.

From the viewpoint of imparting a moisturizing feeling to the skin after drying, the hydrocarbon oil is preferably at least one selected from squalane, squalene, a liquid paraffin, vaseline, and a paraffin wax, and more preferably at least one selected from squalane, vaseline, and a liquid paraffin.

Examples of the cyclic hydrocarbon include fragrances, such as limonene.

Examples of the silicone oil include dimethylpolysiloxane, methylpolysiloxane, methylphenylpolysiloxane, octamethylcyclotetrasiloxane, decamethylcyclopentasiloxane, methylhydrogenpolysiloxane, a silicone resin, an amino-modified silicone, an alkyl-modified silicone, a polyether-modified silicone, a glycerin-modified silicone, and a silicone wax. The silicone oil is preferably one or more selected from these materials, and more preferably dimethylpolysiloxane from the viewpoint of obtaining a dispersion of fine particles. These can also be used as a touch improver or a moisturizing ingredient.

Examples of the dialkyl ether compound include ether compounds having a saturated or unsaturated, linear or branched alkyl group or alkenyl group (preferably having 8 or more and 22 or less carbon atoms). These can also be used as a moisturizing ingredient.

Examples of the amine compound and the amide compound include moisturizing ingredients, such as sphingolipids, e.g., sphingomyelin and ceramide.

Examples of the oils and fats include vegetable oils, such as soybean oil, coconut oil, palm kernel oil, linseed oil, cotton seed oil, rapeseed oil, tung oil, and castor oil. These can also be used as a moisturizing ingredient.

Examples of the higher fatty acid include higher fatty acids having a total carbon number of preferably 8 or more and 30 or less, more preferably 10 or more and 26 or less, and still more preferably 12 or more and 22 or less. Specifically, examples thereof include lauric acid, myristic acid, palmitic acid, stearic acid, behenic acid, oleic acid, linoleic acid, linolenic acid, lanolic acid, and isostearic acid. These can also be used as a moisturizing ingredient.

The oil agent is preferably at least one functional oil agent selected from a refreshing agent, a moisturizing ingredient, a disinfectant, an ultraviolet absorber, and a fragrance.

Examples of the preferred functional oil agent include a moisturizing ingredient, such as N-(2-hydroxy-3-hexadecyloxypropyl)-N-2-hydroxyethyl hexadecanamide (ceramide); an organic ultraviolet absorber, such as 2-ethylhexyl paramethoxycinnamate; a sphingolipid, such as 1-(2-hydroxyethylamino)-3-isostearyloxy-2-propanol; a refreshing agent, such as menthol; a fragrance, such as cedrol; and a disinfectant, such as triclosan and isopropyl methylphenol.

The oil agent may be used alone or may be used in combination of two or more thereof.

### (Water-Insoluble Particles)

The salt-sensitive particles of the present invention may contain water-insoluble particles from the viewpoint of increasing a massage feeling. In particular, in the case of blending the salt-sensitive particles of the present invention in a cleaner, for example, a facial cleaner or a body shampoo, it is preferred that the salt-sensitive particles of the present invention contain water-insoluble particles from the viewpoint of improving the massage feeling.

Here, the wording "water-insoluble" is judged by the fact that when 1 part by mass of the objective particles is dissolved in 99 parts by mass of water at 25°C, less than 50% by mass of the particles are dissolved.

The water-insoluble particles may be used alone or may be used in combination of two or more thereof.

The water-insoluble particles may be organic particles or inorganic particles.

Examples of the water-insoluble organic particles include synthetic polymers, such as polyethylene, polypropylene, a polyamide, polyethylene terephthalate, polystyrene or polyurethane or a crosslinked product thereof, polysodium (meth)acrylate or a poly(meth)acrylic acid ester or a crosslinked product thereof, and besides, a rubber, e.g., an ethylene rubber, a propylene rubber, a styrene-butadiene rubber, a butadiene rubber, and a silicone rubber, or a crosslinked product thereof; natural polymers of derivatives thereof, such as cellulose or a derivative thereof, chitosan or a derivative thereof, a starch, e.g., potato starch and corn starch, and a fruit shell. Here, the term "poly(meth)acrylic acid" means both "polyacrylic acid" and "polymethacrylic acid". Above all, polyethylene, a polyamide, polystyrene, polysodium (meth)acrylate, a poly(meth)acrylic acid ester, cellulose or a derivative thereof, and a starch (preferably corn starch) are preferred, and cellulose or a derivative thereof and a starch (preferably corn starch) are more preferred.

Examples of the water-insoluble inorganic particles include bentonite, talc, mica, kaolin, sepiolite, silica, zeolite, calcium carbonate, titanium oxide, silicic acid anhydride, and hydroxy calcium apatite, and besides, a pearly substance. Above all, bentonite, talc, mica, kaolin, silica, and zeolite are preferred, and bentonite, mica, and silica are more preferred.

As for the water-insoluble particles, a combination of water-insoluble organic particles and water-insoluble inorganic particles can also be used.

The shape of the water-insoluble particles may be a true spherical shape, a substantially spherical shape, or an irregular shape owing to pulverization or the like. In addition, hollow or porous particles can also be used. In the case of using two or more kinds of particles, the shape may be the same as or may be different from each other.

An average particle diameter of the water-insoluble particles is preferably 100 pm or less, and more preferably 70 pm or less from the viewpoint of containing in the salt-sensitive particles, and it is preferably 0.1 pm or more, and more preferably 1 pm or more from the viewpoint of massage feeling.

In this specification, as for the average particle diameter of the water-insoluble particles, the measurement is performed with a laser diffraction/scattering particle size distribution analyzer, LA-920 (manufactured by Horiba, Ltd.), and a median diameter thereof is defined as the average particle diameter.

In the case where the salt-sensitive particles contain the water-insoluble particles, the content of the water-insoluble particles in the salt-sensitive particles is preferably 10% by mass or more, more preferably 20% by mass or more, still more preferably 30% by mass or more, and yet still more preferably 40% by mass or more from the viewpoint of massage feeling, and it is preferably 95% by mass or less, and more preferably 90% by mass or less from the viewpoint of stability of the salt-sensitive particles.

The content of the water-insoluble particles in the salt-sensitive particles is preferably 10% by mass or more and 95% by mass or less, more preferably 20% by mass or more and 95% by mass or less, still more preferably 30% by mass or more and 90% by mass or less, and yet still more preferably 40% by mass or more and 90% by mass or less from the aforementioned viewpoint.

### (Other Components)

The salt-sensitive particles of the present invention may contain other components in addition to the aforementioned components. Examples of the other component include a polymer component other than the acid-modified polyvinyl alcohol, a surfactant, a coloring agent (e.g., a dye and a pigment), an antiseptic, a thickener, and other additives. In addition, a condensing agent, a disinfectant, an ultraviolet absorber, a whitening agent, an anti-inflammatory agent, and the like can be contained. These are contained as the oil agent so far as they are an organic compound having a solubility in 100 g of water of less than 1%.

### <Polymer Other Than Acid-Modified Polyvinyl Alcohol>

In the present invention, in addition to the acid-modified polyvinyl alcohol, other water-soluble polymer than the acid-modified polyvinyl alcohol (the other water-soluble polymer will be hereinafter also referred to as "other polymer") may be contained. Examples of the other polymer component include an unmodified polyvinyl alcohol, a carboxymethyl cellulose, a poly(meth)acrylic acid ester, and a (meth)acrylic acid/(meth)acrylic acid ester copolymer.

### <Surfactant>

In the present invention, it is preferred that the salt-sensitive particles contain a surfactant from the viewpoint of dispersing the oil agent in the acid-modified polyvinyl alcohol. Specifically, examples of the surfactant include an anionic surfactant, a cationic surfactant, a nonionic surfactant, and an amphoteric surfactant. These may be used alone or may be used in combination of two or more thereof.

It is preferred that the surfactant contains at least one selected from an anionic surfactant, an amphoteric surfactant, and a nonionic surfactant from the viewpoint of imparting moderate disintegrability to the salt-sensitive particles and dispersing the oil agent in the salt-sensitive particles. It is more preferred that the surfactant contains an anionic surfactant and a nonionic surfactant from the same viewpoint.

The anionic surfactant is preferably an anionic surfactant having a hydrocarbon group having preferably 12 to 24 carbon atoms, more preferably 12 to 16 carbon atoms, and still more preferably 12 to 14 carbon atoms. Examples thereof include fatty acid salts having 12 to 24 carbon atoms, such as sodium laurate, potassium laurate, and potassium palmitate; polyoxyethylene alkyl ether carboxylic acid salts, such as sodium polyoxyethylene tridecyl ether acetate; alkyl phosphoric acid salts, such as potassium lauryl phosphate, sodium lauryl phosphate, arginine lauryl phosphate, potassium myristyl phosphate, sodium myristyl phosphate, arginine myristyl phosphate, potassium palmityl phosphate, sodium palmityl phosphate, and arginine palmityl phosphate; polyoxyethylene alkyl ether phosphoric acid salts, such as sodium polyoxyethylene oleyl ether phosphate and sodium polyoxyethylene stearyl ether phosphate; alkyl sulfuric acid ester salts, such as sodium lauryl sulfate and potassium lauryl sulfate; polyoxyethylene alkyl ether sulfuric acid ester salts, such as potassium polyoxyethylene lauryl sulfate, sodium polyoxyethylene lauryl sulfate, and polyoxyethylene lauryl sulfate triethanolamine; acylated amino acid salts, such as sodium lauroylsarcosinate, monosodium N-lauroylglutamate, disodium N-stearoylglutamate, monosodium N-myristoyl-L-glutamate, N-lauroylglycine triethanolamine, potassium N-cocoyl glycinate, N-lauroyl-β-alanine triethanolamine, and N-stearoyl-β-aniline triethanolamine; fatty acid amidosulfonic acid salts, such as sodium N-myristoyl-N-methyltaurate and sodium N-stearoyl-N-methyltaurate; and sulfosuccinic acid salts, such as sodium di-2-ethylhexylsulfosuccinate.

Of these anionic surfactants, from the viewpoint of finely dispersing the oil agent in the salt-sensitive particles, at least one selected from the group consisting of fatty acid salts having 12 to 24 carbon atoms, polyoxyethylene alkyl ether carboxylic acid salts, alkyl phosphoric acid salts, polyoxyethylene alkyl ether sulfuric acid ester salts, and acylated amino acid salts is preferred, and at least one selected from the group consisting of fatty acid salts having 12 to 24 carbon atoms, polyoxyethylene alkyl ether carboxylic acid salts, polyoxyethylene alkyl ether sulfuric acid ester salts, and acylated amino acid salts is more preferred,. It is still more preferred to contain a polyoxyethylene alkyl ether carboxylic acid salt and a polyoxyethylene alkyl ether sulfuric acid ester salt.

From the viewpoint of finely dispersing the oil agent in the salt-sensitive particles, the content of the anionic surfactant in the salt-sensitive particles is preferably 0.3 parts by mass or more, more preferably 1 part by mass or more, and still more preferably 3 parts by mass or more, and it is preferably 50 parts by mass or less, more preferably 25 parts by mass or less, and still more preferably 15 parts by mass or less, based on 100 parts by mass of the oil agent. The content of the anionic surfactant in the salt-sensitive particles is preferably 0.3 parts by mass or more and 50 parts by mass or less, more preferably 1 part by mass or more and 25 parts by mass or less, and still more preferably 3 parts by mass or more and 15 parts by mass or less based on 100 parts by mass of the oil agent from the same viewpoint.

Examples of the amphoteric surfactant include betaine-based amphoteric surfactants, such as lauryldimethylaminoacetic acid betaine, lauroylamidobetaine, and laurylsulfobetaine.

Examples of the nonionic surfactant include sorbitan fatty acid esters, such as sorbitan monostearate; polyglycerin fatty acid esters, such as a glycerin fatty acid ester and polyglyceryl monoisostearate; polyoxyethylene fatty acid esters, such as a propylene glycol fatty acid ester and polyethylene glycol monolaurate; sucrose fatty acid esters; polyoxyethylene sorbitan fatty acid esters, such as polyoxyethylene sorbitan monostearate, polyoxyethylene sorbitan monostearate, and polyoxyethylene sorbitan cocoate; polyoxyethylene alkyl ethers; polyoxyethylene sorbitol fatty acid esters; polyoxyethylene glycerin fatty acid esters; polyoxyethylene propylene glycol fatty acid esters; polyoxyethylene castor oil; polyoxyethylene hydrogenated castor oil; polyoxyethylene hydrogenated castor oil fatty acid esters; alkyl polyglucosides; and polyoxyalkylene-modified silicones, such as a polyoxyethylene/methyl polysiloxane copolymer. Of these, at least one selected from the group consisting of sorbitan fatty acid esters, polyoxyethylene sorbitan fatty acid esters, polyoxyethylene fatty acid esters, polyoxyethylene alkyl ethers, polyoxyethylene hydrogenated castor oil, and alkyl polyglucosides is preferred; and at least one selected from the group consisting of sorbitan fatty acid esters, polyoxyethylene sorbitan fatty acid esters, polyoxyethylene fatty acid esters, polyoxyethylene hydrogenated castor oil, and alkyl polyglucosides is more preferred. It is still more preferred to contain a polyoxyethylene sorbitan fatty acid ester.

From the viewpoint of finely dispersing the oil agent in the salt-sensitive particles, the content of the nonionic surfactant in the salt-sensitive particles is preferably 0.3 parts by mass or more, more preferably 1 part by mass or more, and still more preferably 3 parts by mass or more, and it is preferably 50 parts by mass or less, more preferably 25 parts by mass or less, and still more preferably 15 parts by mass or less, based on 100 parts by mass of the oil agent. The content of the nonionic surfactant in the salt-sensitive particles is preferably 0.3 parts by mass or more and 50 parts by mass or less, more preferably 1 part by mass or more and 25 parts by mass or less, and still more preferably 3 parts by mass or more and 15 parts by mass or less based on 100 parts by mass of the oil agent from the same viewpoint.

From the viewpoint of finely dispersing the oil agent in the salt-sensitive particles, the total content of the surfactant in the salt-sensitive particles is preferably 0.6 parts by mass or more, more preferably 2 parts by mass or more, and still more preferably 6 parts by mass or more, and it is preferably 100 parts by mass or less, more preferably 50 parts by mass or less, and still more preferably 35 parts by mass or less, based on 100 parts by mass of the oil agent. The total content of the surfactant in the salt-sensitive particles is preferably 0.6 parts by mass or more and 100 parts by mass or less, more preferably 2 parts by mass or more and 50 parts by mass or less, and still more preferably 6 parts by mass or more and 35 parts by mass or less based on 100 parts by mass of the oil agent.

### <Other Additives>

The salt-sensitive particles of the present invention may contain other additives in addition to the aforementioned components in order to immobilize the dispersed oil agent inside the particles. Other additives may have shaping and increasing effects. Examples thereof include monosaccharides, disaccharides, and polysaccharides, such as glucose, fructose, lactose, maltose, sucrose, dextrin, maltodextrin, cyclodextrin, maltose, fructose, and trehalose; sugar alcohols, such as sorbitol, mannitol, maltitol, lactose, maltotriitol, xylitol, and a polyhydric alcohol; polysaccharide thickeners, such as gum arabic, guar gum, pectin, pullulan, and sodium alginate; cellulose derivatives, such as methyl cellulose; and starch derivatives obtained by subjecting starch to esterification treatment, etherification treatment, or terminal reduction treatment; and besides, processed starches, gelatin decomposition products, and agar. In addition, the other additives may be used in combination of two or more thereof as the need arises.

### (Salt-Sensitive Particles)

As for the salt-sensitive particles of the present invention, in the case of blending the salt-sensitive particles in a product, such as a cleaner, since the concentration of the water-soluble salt is high in the product, the particles are not disintegrated, and the oil agent stably exists in the particles. On the other hand, when the concentration of the water-soluble salt in the product is decreased in the cleaning process and the rinsing process, the solubility in water is improved, the particles are disintegrated, and the oil agent is released.

Taking into consideration blending of such a cleaner, as for the salt-sensitive particles of the present invention, a release rate (S₁₀) of the oil agent in 10% by mass salt water is preferably 40% or less, more preferably 20% or less, still more preferably 18% or less, and yet still more preferably 15% or less in terms of a salt concentration from the viewpoint of storage stability of the oil agent in the salt-sensitive particles, and it may also be 0%, and preferably 0% or more.

A release rate (S₁) of the oil agent in 1% by mass salt water is preferably 30% or more, more preferably 35% or more, still more preferably 40% or more, and yet still more preferably 50% or more from the viewpoint of improving the feeling of effects of the oil agent, and it may also be 100%, and more preferably 100% or less.

As for the salt-sensitive particles of the present invention, from the aforementioned viewpoint, preferably, not only the release rate of the oil agent in 10% by mass salt water is 20% or less, but also the release rate of the oil agent in 1% by mass salt water is 30% or more; more preferably, not only the release rate of the oil agent in 10% by mass salt water is 18% or less, but also the release rate of the oil agent in 1% by mass salt water is 35% or more; and still more preferably, not only the release rate of the oil agent in 10% by mass salt water is 15% or less, but also the release rate of the oil agent in 1% by mass salt water is 40% or more.

The aforementioned release rate is a release rate relative to salt water at 25°C and can be determined by the method described in the section of Examples.

In this specification, as for the salt-sensitive particles, when the release rate of the oil agent in 10% by mass salt water is defined as S₁₀%, and the release rate of the oil agent in 1% by mass salt water is defined as S₁%, (S₁ - S₁₀) is preferably 12% or more, more preferably 15% or more, still more preferably 20% or more, and yet still more preferably 25% or more from the viewpoint of excellent salt sensitivity, and it is preferably 80% or less, more preferably 70% or less, and still more preferably 60% or less from the viewpoint of design. From such viewpoint, the (S₁ - S₁₀) is preferably 12% or more and 80% or less, more preferably 15% or more and 70% or less, still more preferably 20% or more and 60% or less, and yet still more preferably 25% or more and 60% or less.

These release rates can be appropriately regulated according to the acid modification rate, the degree of saponification, the molecular weight, and the degree of polymerization of the acid-modified polyvinyl alcohol to be used in the present invention.

For example, by reducing the acid modification rate, the release rate of the oil agent from the salt-sensitive particles in the 10% salt water can be suppressed, and the storage stability of the particles can be improved. In addition, by improving the acid modification rate, the release of the oil agent from the salt-sensitive particles in the 1% salt water can be promoted, and the feeling of effects of the oil agent can be enhanced.

Similarly, by increasing the degree of saponification, the release rate of the oil agent from the salt-sensitive particles in the 10% salt water can be suppressed, and the storage stability of the particles can be improved. In addition, by decreasing the degree of saponification, the release of the oil agent from the salt-sensitive particles in the 1% salt water can be promoted, and the feeling of effects of the oil agent can be enhanced.

Furthermore, by increasing the molecular weight or the degree of polymerization, the release rate of the oil agent from the salt-sensitive particles in the 10% salt water can be suppressed, and the storage stability of the particles can be improved. In addition, by decreasing the molecular weight or the degree of polymerization, the release of the oil agent from the salt-sensitive particles in the 1% salt water can be promoted, and the feeling of effects of the oil agent can be enhanced.

An average dispersion diameter of the oil agent in the salt-sensitive particles of the present invention is preferably 30 pm or less, more preferably 15 pm or less, still more preferably 10 µm, yet still more preferably 3 pm or less, and even yet still more preferably 1 pm or less from the viewpoint of improving the feeling of effects at the time of use on the basis of the oil agent and the viewpoint of improving the yield at the time of production, and it is preferably 0.01 pm or more, more preferably 0.03 pm or more, still more preferably 0.05 pm or more, yet still more preferably 0.07 pm or more, and even yet still more preferably 0.1 pm or more from the viewpoint of easiness of production. The average dispersion diameter of the oil agent in the particles can be measured according to the size of voids in the salt-sensitive particles and can be measured by the method described in the section of Examples.

From the aforementioned viewpoint, the average dispersion diameter of the oil agent in the salt-sensitive particles of the present invention is 0.01 pm or more and 30 pm or less, more preferably 0.03 pm or more and 15 pm or less, still more preferably 0.05 pm or more and 10 pm or less, yet still more preferably 0.07 pm or more and 3 pm or less, and even yet still more preferably 0.1 pm or more and 1 pm or less.

By reducing the size of emulsified droplets at the time of production as mentioned later, the average particle diameter of the oil agent in the salt-sensitive particles after removing the water can be reduced.

The content of the acid-modified polyvinyl alcohol in the salt-sensitive particles is preferably 1% by mass or more, more preferably 5% by mass or more, still more preferably 10% by mass or more, yet still more preferably 20% by mass or more, and even yet still more preferably 40% by mass or more from the viewpoint of exhibiting the effect of salt sensitivity, and it is preferably 99% by mass or less, more preferably 95% by mass or less, still more preferably 90% by mass or less, yet still more preferably 85% by mass or less, and even yet still more preferably 80% by mass or less from the viewpoint of containing other components, such as the oil agent.

From the aforementioned viewpoint, the content of the acid-modified polyvinyl alcohol in the salt-sensitive particles is preferably 1% by mass or more and 99% by mass or less, more preferably 5% by mass or more and 95% by mass or less, still more preferably 10% by mass or more and 90% by mass or less, yet still more preferably 20% by mass or more and 85% by mass or less, and even yet still more preferably 40% by mass or more and 80% by mass or less.

The acid-modified polyvinyl alcohol forms a matrix of the salt-sensitive particles. That is, the matrix is a polymer component corresponding to a sea portion of the sea-island. The polymer component may contain the aforementioned other components and other additives in addition to the acid-modified polyvinyl alcohol. From the viewpoint of providing favorable salt sensitivity, the content of the acid-modified polyvinyl alcohol in the whole of the polymer component is preferably 50% by mass or more, more preferably 70% by mass or more, still more preferably 90% by mass or more, and yet still more preferably 95% by mass or more and it may also be 100% by mass.

The content of the oil agent in the salt-sensitive particles is preferably 0.1% by mass or more, more preferably 0.5% by mass or more, still more preferably 1% by mass or more, yet still more preferably 5% by mass or more, and even yet still more preferably 10% by mass or more from the viewpoint of exhibiting the effects of the oil agent, and it is preferably 80% by mass or less, more preferably 70% by mass or less, still more preferably 60% by mass or less, yet still more preferably 50% by mass or less, and even yet still more preferably 40% by mass or less from the viewpoint of suppressing the leakage of the oil agent from the salt-sensitive particles.

From the aforementioned viewpoint, the content of the oil agent in the salt-sensitive particles is preferably 0.1% by mass or more and 80% by mass or less, more preferably 0.5% by mass or more and 70% by mass or less, still more preferably 1% by mass or more and 60% by mass or less, yet still more preferably 5% by mass or more and 50% by mass or less, and even yet still more preferably 10% by mass or more and 40% by mass or less.

In the salt-sensitive particles, a mass ratio [(acid-modified polyvinyl alcohol)/(oil agent)] of the content of the acid-modified polyvinyl alcohol to the content of the oil agent is preferably 0.1 or more, more preferably 0.3 or more, still more preferably 0.5 or more, yet still more preferably 0.7 or more, and even yet still more preferably 1 or more from the viewpoint of suppressing the leakage of the oil agent from the salt-sensitive particles, and it is preferably 90 or less, more preferably 70 or less, still more preferably 50 or less, yet still more preferably 30 or less, and even yet still more preferably 10 or less from the viewpoint of efficiently containing the oil agent.

From the aforementioned viewpoint of the mass ratio of the acid-modified polyvinyl alcohol to the oil agent in the salt-sensitive particles is preferably 0.1 or more and 90 or less, more preferably 0.3 or more and 70 or less, still more preferably 0.5 or more and 50 or less, yet still more preferably 0.7 or more and 30 or less, and even yet still more preferably 1 or more and 10 or less.

An average particle diameter (properties of dry product) of the salt-sensitive particles of the present invention is preferably 1,500 pm or less, more preferably 1,000 pm or less, still more preferably 500 pm or less, yet still more preferably 200 pm or less, and even yet still more preferably 100 pm or less from the viewpoint of feeling of use when blended in the cleaner, and it is preferably 1 pm or more, more preferably 5 pm or more, still more preferably 10 pm or more, yet still more preferably 20 pm or more, and even yet still more preferably 30 pm or more from the viewpoint of containing the oil agent.

From the aforementioned viewpoint, the average particle diameter (properties of dry product) of the salt-sensitive particles of the present invention is preferably 1 pm or more and 1,500 pm or less, more preferably 5 pm or more and 1,000 pm or less, still more preferably 10 pm or more and 500 pm or less, yet still more preferably 20 pm or more and 200 pm or less, and even yet still more preferably 30 pm or more and 100 pm or less.

In this specification, as for the average particle diameter of the salt-sensitive particles, the measurement is performed with a laser diffraction/scattering particle size distribution analyzer, LA-920 (manufactured by Horiba, Ltd.) as described in the section of Examples, and a median diameter thereof is defined as the average particle diameter.

A ratio of the average dispersion diameter of the oil agent to the average particle diameter of the salt-sensitive particles [(average dispersion diameter of oil agent)/(average particle diameter of salt-sensitive particles)] is preferably 0.3 or less, more preferably 0.2 or less, still more preferably 0.1 or less, yet still more preferably 0.07 or less, even yet still more preferably 0.04 or less, and even still more preferably 0.02 or less from the viewpoint of finely dispersing the oil agent to improve the feeling of effects on the basis of the oil agent at the time of use and the viewpoint of improving the yield at the time of production, and it is preferably 0.0005 or more, and more preferably 0.001 or more from the viewpoint of easiness of production.

From the aforementioned viewpoint, the ratio of the average dispersion diameter of the oil agent to the average particle diameter of the salt-sensitive particles [(average dispersion diameter of oil agent)/(average particle diameter of salt-sensitive particles)] is preferably 0.0005 or more and 0.3 or less, more preferably 0.0005 or more and 0.2 or less, still more preferably 0.0005 or more and 0.1 or less, yet still more preferably 0.001 or more and 0.1 or less, even yet still more preferably 0.001 or more and 0.07 or less, even still more preferably 0.001 or more and 0.04 or less, and even still more further preferably 0.001 or more and 0.02 or less.

### (Production Method)

As for the method for producing the salt-sensitive particles of the present invention, specifically, the salt-sensitive particles can be obtained by removing water from an emulsion composition containing the oil agent and the acid-modified polyvinyl alcohol.

That is, the method for producing the salt-sensitive particles of the present invention preferably includes the following step 1 and step 2 in this order.
Step 1: A step of preparing an emulsion composition containing an oil agent having a solubility in 100 g of water of less than 1 g and an acid-modified polyvinyl alcohol.
Step 2: A step of removing water from the emulsion composition.

The step 1 preferably includes the following step 1-1 to step 1-3.
Step 1- 1: A step of preparing an oil phase containing the oil agent.
Step 1-2: A step of preparing a water phase containing the acid-modified polyvinyl alcohol.
Step 1-3: A step of mixing two liquids prepared in the step 1-1 and the step 1-2, to obtain the emulsion composition.

The step 1-1 is a step of preparing an oil phase containing the oil agent.

In the step 1-1, though the oil phase may be prepared by heating and melting the oil agent alone, it is preferred to prepare the dissolved or dispersed oil phase by adding the oil agent with a surfactant and further, optionally with other components, such as an emulsification adjuvant.

In the step 1-2, the acid-modified polyvinyl alcohol and water and optionally, other components are mixed/dissolved to prepare the water phase.

In the step 1-3, it is preferred to prepare the emulsion composition by mixing the previously prepared oil phase component and the water phase component under stirring.

An emulsion diameter is preferably 100 pm or less, more preferably 50 pm or less, still more preferably 30 µm or less, yet still more preferably 10 pm or less, even yet still more preferably 5 pm or less, and even still more preferably 1 pm or less from the viewpoint of improving a residual rate of the oil agent, the viewpoint of improving the yield at the time of production, and the viewpoint of reducing the average particle diameter of the oil agent in the obtained salt-sensitive particles to exhibit the effects of the oil agent. In addition, the emulsion diameter is preferably 0.01 pm or more, more preferably 0.05 pm or more, and still more preferably 0.1 pm or more from the viewpoint of easiness of production.

From the aforementioned viewpoint, the emulsion diameter is preferably 0.01 pm or more and 100 pm or less, more preferably 0.05 pm or more and 50 pm or less, still more preferably 0.1 pm or more and 30 pm or less, yet still more preferably 0.1 pm or more and 10 pm or less, even yet still more preferably 0.1 pm or more and 5 pm or less, and even still more preferably 0.1 pm or more and 1 pm or less.

As for an emulsifier, a static emulsifier/disperser, a general stirrer, such as a propeller blade and a flat plate blade, a stirring type emulsifier, such as a homomixer and a disper mixer, and a high-pressure emulsifier, such as a homogenizer and a nanomizer, are preferably used.

On the occasion of emulsification, it is preferred to use the aforementioned surfactant together with the oil agent from the viewpoint of reducing the emulsion diameter and finely dispersing the oil agent in the salt-sensitive particles. As for the surfactant, there is preferably exemplified an anionic surfactant or a nonionic surfactant. The preferred content of the surfactant based on 100 parts by mass of the oil agent is as mentioned above.

In the case where the salt-sensitive particles contain water-insoluble particles, it is preferred that after preparing the emulsion composition in the step 1-3, the water-insoluble particles are added to the emulsion composition.

The step 2 is a step of removing water from the emulsion composition prepared in the step 1.

Examples of a method for removing water include spray drying, freeze drying, vacuum drying, belt drying, shelf drying, and drum drying. Of these, the spray drying method is preferred from the viewpoint of easiness of regulation of the particle shape and storage stability of the oil agent in the salt-sensitive particles. In the case of performing the drying by other method than the spray drying method, pulverization is performed in order to obtain the particles having a desired particle diameter, as the need arises.

Examples of the granulation method include granulation methods, such as tumbling granulation, tumbling fluidized granulation, fluidized bed granulation, and stirring tumbling granulation.

In the case of using the water-insoluble particles, the water-insoluble particles can also be contained in the emulsion composition through addition thereof in the step 1. Alternatively, a step 1' of mixing the emulsion composition obtained in the step 1 and the water-insoluble particles, followed by granulation can be adopted, too.

In the case where the granulation product has been obtained in the step 1', freeze drying, vacuum drying, shelf drying, or the like is preferably exemplified.

### (Cleaner)

The salt-sensitive particles produced by the method of the present invention can be widely used for various products, for example, a skin cleaner, such as a facial cleaner, a body cleaner, and a solid soap; a hair cleaner, such as a shampoo; a dentifrice; a cleaner for tableware; a cleaner for clothing; a softener for clothing; and a cleaner for contact lens. Above all, the salt-sensitive particles produced by the method of the present invention are suitably used for cleaners, and especially suitably used for cleaners for cleaning a skin, a hair, or a clothing. Of these, the cleaner is useful as a cosmetic, and especially useful as a skin cleaner.

The content of the salt-sensitive particles in the product having the salt-sensitive particles blended therein, such as the cleaner of the present invention, is preferably 0.1% by mass or more, more preferably 0.5% by mass or more, and still more preferably 1% by mass or more from the viewpoint of exhibiting the effects of the oil agent, and it is preferably 30% by mass or less, more preferably 20% by mass or less, and still more preferably 10% by mass or less from the viewpoint of product costs. From the aforementioned viewpoint, the content of the salt-sensitive particles in the product having the salt-sensitive particles blended therein is preferably 0.1% by mass or more and 30% by mass or less, more preferably 0.5% by mass or more and 20% by mass or less, and still more preferably 1% by mass or more and 10% by mass or less.

From the viewpoint of cleaning properties, the total content of the surfactant in the cleaner of the present invention is preferably 0.1% by mass or more, more preferably 0.3% by mass or more, and still more preferably 0.5% by mass or more, and it is preferably 50% by mass or less, more preferably 45% by mass or less, still more preferably 40% by mass or less, and yet still more preferably 35% by mass or less.

Examples of the surfactant include the aforementioned anionic surfactants and salts thereof, the aforementioned amphoteric surfactants, and the aforementioned nonionic surfactants. Examples thereof include anionic surfactants, such as a fatty acid soap, a phosphoric acid ester, an acylated amino acid, a sulfosuccinic acid, a taurate-based active agent, a polyoxyethylene alkyl sulfuric acid salt, and a polyoxyethylene alkyl ether carboxylic acid or a salt thereof; and nonionic surfactants, such as an alkyl saccharide and an EO-adduct type surfactant, e.g., a polyoxyethylene alkyl ether.

From the viewpoint of storage stability of the salt-sensitive particles in the cleaner, it is preferred that the cleaner containing the salt-sensitive particles contains an anionic surfactant or a salt thereof, an amphoteric surfactant, an inorganic salt, an organic salt, a basic amino acid, and a basic amine.

From the viewpoint of storage stability of the salt-sensitive particles in the cleaner, the total content of the anionic surfactant or its salt, the amphoteric surfactant, the inorganic salt, the organic salt, the basic amino acid, and the basic amine in the cleaner is preferably 0.5% by mass or more, more preferably 1% by mass or more, still more preferably 3% by mass or more, yet still more preferably 5% by mass or more, and even yet still more preferably 10% by mass or more, and it is preferably 50% by mass or less, more preferably 45% by mass or less, still more preferably 40% by mass or less, and yet still more preferably 35% by mass or less.

Examples of the surfactant include the aforementioned anionic surfactant and amphoteric surfactant.

The inorganic salt is preferably a water-soluble inorganic salt, and examples thereof include chlorides, such as sodium chloride, potassium chloride, and magnesium chloride; sulfates, such as sodium sulfate, potassium sulfate, magnesium sulfate, and aluminum sulfate; and carbonates, such as sodium carbonate and sodium hydrogencarbonate. In the case of sodium chloride, generally marketed common salt, high-purity refined salt, and natural salt, and so on are used. Above all, sodium chloride, potassium chloride, magnesium chloride, and sodium carbonate are especially preferably used.

As for the organic salt, there are preferably exemplified organic acid salts having 2 to 6 carbon atoms, such as a citric acid salt, a malic acid salt, and a maleic acid salt. Examples of the salt include alkali metal salts of sodium, potassium, or the like; and alkaline earth metal salts.

Examples of the basic amine include tromethamine, triethanolamine, diethanolamine, monoethanolamine, glucosamine, galactosamine, fructosamine, meglumine, and N-ethylglucamine; and examples of the basic amino acid include lysine, arginine, histidine, tryptophan, and ornithine. The basic amine or basic amino acid may work as a counter ion of the aforementioned anionic surfactant or organic acid.

A pH of the cleaner containing the salt-sensitive particles of the present invention is preferably 3 to 9, more preferably 4 to 8, and still more preferably 5 to 7 from the viewpoint of stability of the salt-sensitive particles.

### (Cleaning Method)

In the present invention, the aforementioned cleaner is preferably used for a method for cleaning a clothing or a method for cleaning a skin or hair.

Preferably, the method for cleaning a skin or hair according to the present invention is a method of using the cleaner containing the salt-sensitive particles of the present invention.

In consequence, the cleaner is preferably a skin cleaner, such as a facial cleaner and a body shampoo, or a hair cleaner, such as a shampoo. The skin cleaner and the hair cleaner are each preferably diluted about 2 to 30 times at the time of cleaning, and a physical force, such as massage, is applied along with a reduction of the concentration of the water-soluble salt, and therefore, the salt-sensitive particles are readily disintegrated. In addition, the salt concentration is further decreased at the time of rinsing, and the salt-sensitive particles are readily disintegrated.

The present invention further discloses the following <1> to <35>.
<1> Salt-sensitive particles containing an oil agent having a solubility in 100 g of water of less than 1 g and an acid-modified polyvinyl alcohol, wherein the oil agent is dispersed in the salt-sensitive particles.
<2> The salt-sensitive particles as set forth in <1>, wherein the acid-modified polyvinyl alcohol is preferably an acid-modified polyvinyl alcohol having at least one of a sulfonic acid group and a carboxylic acid group introduced thereinto, and more preferably an acid-modified polyvinyl alcohol having a carboxylic acid group introduced thereinto.
<3> The salt-sensitive particles as set forth in <1> or <2>, wherein an acid modification rate in the acid-modified polyvinyl alcohol is preferably 0.1 mol% or more and 10 mol% or less, more preferably 0.5 mol% or more and 5 mol% or less, and still more 1 mol% or more and 3 mol% or less.
<4> The salt-sensitive particles as set forth in any of <1> to <3>, wherein a degree of saponification of the acid-modified polyvinyl alcohol is preferably 70 mol% or more and 99.9 mol% or less, more preferably 80 mol% or more and 99.5 mol% or less, and still more preferably 90 mol% or more and 99 mol% or less.
<5> The salt-sensitive particles as set forth in any of <1> to <4>, wherein a degree of polymerization of the acid-modified polyvinyl alcohol is preferably 100 or more and 200,000 or less, more preferably 500 or more and 10,000 or less, and still more preferably 1,000 or more and 4,000 or less.
<6> The salt-sensitive particles as set forth in any of <1> to <5>, wherein the solubility of the oil agent in 100 g of water at 25°C is preferably 0 g or more and less than 1 g, more preferably 0 g or more and 0.5 g or less, still more preferably 0 g or more and 0.3 g or less, and yet still more preferably 0 g or more and 0.1 g or less.
<7> The salt-sensitive particles as set forth in any of <1> to <6>, wherein a melting point of the oil agent is -100°C or higher and lower than 100°C, more preferably 0°C or higher and 99°C or lower, still more preferably 0°C or higher and 95°C or lower, yet still more preferably 10°C or higher and 90°C or lower, even yet still more preferably 10°C or higher and 80°C or lower, even still more preferably 20°C or higher and 70°C or lower, and even still more further preferably 20°C or higher and 60°C or lower.
<8> The salt-sensitive particles as set forth in any of <1> to <7>, wherein a molecular weight of the oil agent is preferably 80 or more and 10,000 or less, more preferably 100 or more and 6,000 or less, still more preferably 100 or more and 1,000 or less, and yet still more preferably 100 or more and 500 or less.
<9> The salt-sensitive particles as set forth in any of <1> to <8>, wherein the oil agent is at least one selected from an alcohol, an ester oil, a hydrocarbon oil, a silicone oil, a dialkyl ether compound, an amine compound, an amide compound, oils and fats, and a higher fatty acid.
<10> The salt-sensitive particles as set forth in any of <1> to <9>, wherein the oil agent is at least one functional oil agent selected from a refreshing agent, a moisturizing ingredient, a disinfectant, an ultraviolet absorber, and a fragrance.
<11> The salt-sensitive particles as set forth in any of <1> to <10>, further containing a surfactant.
<12> The salt-containing particles as set forth in <11>, wherein the total content of the surfactant in the salt-sensitive particles is preferably 0.6 parts by mass or more and 100 parts by mass or less, more preferably 2 parts by mass or more and 50 parts by mass or less, and still more preferably 6 parts by mass or more and 35 parts by mass or less based on 100 parts by mass of the oil agent.
<13> The salt-sensitive particles as set forth in any of <1> to <12>, wherein in the salt-sensitive particles, a release rate (S₁₀) of the oil agent in 10% by mass salt water is preferably 40% or less, more preferably 20% or less, still more preferably 18% or less, and yet still more preferably 15% or less, and it may also be 0%, and preferably 0% or more.
<14> The salt-sensitive particles as set forth in any of <1> to <13>, wherein in the salt-sensitive particles, a release rate (S₁) of the oil agent in 1% by mass salt water is preferably 30% or more, more preferably 35% or more, still more preferably 40% or more, and yet still more preferably 50% or more, and it may also be 100%, and more preferably 100% or less.
<15> The salt-sensitive particles as set forth in any of <1> to <14>, wherein in the salt-sensitive particles, preferably, not only the release rate of the oil agent in 10% by mass salt water is 20% or less, but also the release rate of the oil agent in 1% by mass salt water is 30% or more; more preferably, not only the release rate of the oil agent in 10% by mass salt water is 18% or less, but also the release rate of the oil agent in 1% by mass salt water is 35% or more; and still more preferably, not only the release rate of the oil agent in 10% by mass salt water is 15% or less, but also the release rate of the oil agent in 1% by mass salt water is 40% or more.
<16> The salt-sensitive particles as set forth in any of <1> to <15>, wherein in the salt-sensitive particles, a difference (S₁ - S₁₀) between the release rate (S₁₀) of the oil agent in 10% by mass salt water and the release rate (S₁) of the oil agent in 1% by mass salt water is preferably 12% or more, more preferably 15% or more, still more preferably 20% or more, and yet still more preferably 25% or more, and it is preferably 80% or less, more preferably 70% or less, and still more preferably 60% or less.
<17> The salt-sensitive particles as set forth in any of <1> to <16>, wherein in the salt-sensitive particles, a difference (S₁ - S₁₀) between the release rate (S₁₀) of the oil agent in 10% by mass salt water and the release rate (S₁) of the oil agent in 1% by mass salt water is preferably 12% or more and 80% or less, more preferably 15% or more and 70% or less, still more preferably 20% or more and 60% or less, and yet still more preferably 25% or more and 60% or less.
<18> The salt-sensitive particles as set forth in any of <1> to <17>, wherein an average dispersion diameter of the oil agent in the salt-sensitive particles is 0.01 µm or more and 30 pm or less, more preferably 0.03 pm or more and 15 pm or less, still more preferably 0.05 pm or more and 10 pm or less, yet still more preferably 0.07 pm or more and 3 pm or less, and even yet still more preferably 0.1 pm or more and 1 µm or less.
<19> The salt-sensitive particles as set forth in any of <1> to <18>, wherein the content of the acid-modified polyvinyl alcohol in the salt-sensitive particles is preferably 1% by mass or more, more preferably 5% by mass or more, still more preferably 10% by mass or more, yet still more preferably 20% by mass or more, and even yet still more preferably 40% by mass or more, and it is preferably 99% by mass or less, more preferably 95% by mass or less, still more preferably 90% by mass or less, yet still more preferably 85% by mass or less, and even yet still more preferably 80% by mass or less.
<20> The salt-sensitive particles as set forth in any of <1> to <19>, wherein the content of the acid-modified polyvinyl alcohol in the salt-sensitive particles is preferably 1% by mass or more and 99% by mass or less, more preferably 5% by mass or more and 95% by mass or less, still more preferably 10% by mass or more and 90% by mass or less, yet still more preferably 20% by mass or more and 85% by mass or less, and even yet still more preferably 40% by mass or more and 80% by mass or less.
<21> The salt-sensitive particles as set forth in any of <1> to <20>, wherein the content of the oil agent in the salt-sensitive particles is preferably 0.1% by mass or more, more preferably 0.5% by mass or more, still more preferably 1% by mass or more, yet still more preferably 5% by mass or more, and even yet still more preferably 10% by mass or more, and it is preferably 80% by mass or less, more preferably 70% by mass or less, still more preferably 60% by mass or less, yet still more preferably 50% by mass or less, and even yet still more preferably 40% by mass or less.
<22> The salt-sensitive particles as set forth in any of <1> to <21>, wherein the content of the oil agent in the salt-sensitive particles is preferably 0.1% by mass or more and 80% by mass or less, more preferably 0.5% by mass or more and 70% by mass or less, still more preferably 1% by mass or more and 60% by mass or less, yet still more preferably 5% by mass or more and 50% by mass or less, and even yet still more preferably 10% by mass or more and 40% by mass or less.
<23> The salt-sensitive particles as set forth in any of <1> to <22>, wherein a mass ratio of the acid-modified polyvinyl alcohol to the oil agent in the salt-sensitive particles is preferably 0.1 or more and 90 or less, more preferably 0.3 or more and 70 or less, still more preferably 0.5 or more and 50 or less, yet still more preferably 0.7 or more and 30 or less, and even yet still more preferably 1 or more and 10 or less.
<24> The salt-sensitive particles as set forth in any of <1> to <23>, wherein an average particle diameter of the salt-sensitive particles is preferably 1 pm or more and 1,500 pm or less, more preferably 5 pm or more and 1,000 pm or less, still more preferably 10 pm or more and 500 pm or less, yet still more preferably 20 pm or more and 200 pm or less, and even yet still more preferably 30 pm or more and 100 pm or less.
<25> The salt-sensitive particles as set forth in any of <1> to <24>, wherein a ratio of the average dispersion diameter of the oil agent to the average particle diameter of the salt-sensitive particles [(average dispersion diameter of oil agent)/(average particle diameter of salt-sensitive particles)] is preferably 0.0005 or more and 0.3 or less, more preferably 0.0005 or more and 0.2 or less, still more preferably 0.0005 or more and 0.1 or less, yet still more preferably 0.001 or more and 0.1 or less, even yet still more preferably 0.001 or more and 0.07 or less, even still more preferably 0.001 or more and 0.04 or less, and even still more further preferably 0.001 or more and 0.02 or less.
<26> A method for producing the salt-sensitive particles as set forth in any of <1> to <25>, including the following step 1 and step 2 in this order:
   Step 1: a step of preparing an emulsion composition containing an oil agent having a solubility in 100 g of water of less than 1 g and an acid-modified polyvinyl alcohol; and
   Step 2: a step of removing water from the emulsion composition.
<27> The method for producing the salt-sensitive particles as set forth in <26>, wherein the step 1 preferably includes the following step 1-1 to step 1-3:
   Step 1- 1: a step of preparing an oil phase containing the oil agent;
   Step 1-2: a step of preparing a water phase containing the acid-modified polyvinyl alcohol; and
   Step 1-3: a step of mixing two liquids prepared in the step 1-1 and the step 1-2, to obtain the emulsion composition.
<28> The method for producing the salt-sensitive particles as set forth in <26> or <27>, wherein an emulsion diameter of the emulsion composition is preferably 0.01 pm or more and 100 pm or less, more preferably 0.05 pm or more and 50 pm or less, still more preferably 0.1 pm or more and 30 pm or less, yet still more preferably 0.1 pm or more and 10 pm or less, even yet still more preferably 0.1 pm or more and 5 pm or less, and even still more preferably 0.1 pm or more and 1 pm or less.
<29> A product containing the salt-sensitive particles as set forth in any of <1> to <25>, inclusive of a skin cleaner, such as a facial cleaner, a body cleaner, and a solid soap; a hair cleaner, such as a shampoo; a dentifrice; a cleaner for tableware; a cleaner for clothing; a softener for clothing; and a cleaner for contact lens.
<30> The product as set forth in <29>, wherein the product is a cleaner, and preferably a skin cleaner.
<31> A product, such as a cleaner having the salt-sensitive particles as set forth in any of <1> to <25> blended therein, wherein the content of the salt-sensitive particles in the product is preferably 0.1% by mass or more, more preferably 0.5% by mass or more, and still more preferably 1% by mass or more, and it is preferably 30% by mass or less, more preferably 20% by mass or less, and still more preferably 10% by mass or less.
<32> A product, such as a cleaner having the salt-sensitive particles as set forth in any of <1> to <25> blended therein, wherein the content of the salt-sensitive particles in the product is preferably 0.1% by mass or more and 30% by mass or less, more preferably 0.5% by mass or more and 20% by mass or less, and still more preferably 1% by mass or more and 10% by mass or less.
<33> The product, such as a cleaner, as set forth in any of <29> to <32>, wherein the product contains a surfactant, and the total content of the surfactant is preferably 0.1% by mass or more, more preferably 0.3% by mass or more, and still more preferably 0.5% by mass or more, and it is preferably 50% by mass or less, more preferably 45% by mass or less, still more preferably 40% by mass or less, and yet still more preferably 35% by mass or less.
<34> A cleaner containing the salt-sensitive particles as set forth in any of <1> to <25>.
<35> A method for cleaning a skin, a hair, or a clothing, including using the cleaner as set forth in <34>.

### Examples

In the following Examples, the term "%" means "% by mass".

### [Analysis Method]

### (i) Measurement of emulsion average particle diameter and average particle diameters of water-insoluble particles and salt-sensitive particles

As for the measurement of the emulsion average particle diameter and the average particle diameter of the water-insoluble particles, a median diameter measured with a laser diffraction/scattering particle size distribution analyzer, LA-920 (manufactured by Horiba, Ltd.) by dispersing in ion-exchanged water was defined as the average particle diameter. A measurement temperature was 25°C, and a relative refractive index was 1.2.

In addition, as for the measurement of the obtained salt-sensitive particles, a median diameter measured with a laser diffraction/scattering particle size distribution analyzer, LA-920 (manufactured by Horiba, Ltd.) by preliminarily dispersing in a small amount of propylene glycol (manufactured by Wako Pure Chemical Industries, Ltd.) and then dispersing in 20% by mass salt water under the same condition as mentioned above was defined as the average particle diameter.

### [Measurement of Average Particle Diameter of Salt-Sensitive Particles of Example 16]

Using 12-step sieves of 2,000 µm, 1,400 µm, 1,000 µm, 710 µm, 500 µm, 355 µm, 250 µm, 180 µm, 125 µm, 90 µm, 63 µm, and 45 pm as prescribed in JIS Z8801-1 (established on May 20, 2000 and last revised on November 20, 2006) and a receiving tray, the sieves were stacked in the order from the smallest opening on the receiving tray; 100 g of the particles were added from the top of the uppermost 2,000 µm-sieve; after lidding, the resultant was installed in a low-tap type sieve shaker (manufactured by HEIKO SEISAKUSHO, Ltd., tapping: 156 times/min, rolling: 290 times/min); and after vibrating for 5 minutes, the masses of the particles remaining on each of the sieves and the receiving tray were measured, thereby calculating a mass proportion (%) of the particles on each sieve. The mass proportions of the particles in the order beginning from the receiving tray to those sieves having smaller sieve openings were cumulated, and a particle diameter at which a total was 50% was defined as the average particle diameter.

### (ii) Measurement method of menthol residual rate

About 0.03 g of the obtained salt-sensitive particles were weighed (weighed 0.2 g in the case of the particles of Example 13) and preliminarily dispersed in 0.2 mL of propylene glycol (manufactured by Wako Pure Chemical Industries, Ltd.); 5 mL of a mixed solution of ion-exchanged water/DMSO (dimethyl sulfoxide, manufactured by Wako Pure Chemical Industries, Ltd.) (1/1 vol/vol) was then added; and the resultant was treated with an ultrasonic cleaning machine (manufactured by SND Co., Ltd., US-4, frequency: 38 kHz, 50°C) until the particles were dissolved.

To the aforementioned solution, 2 g of NaCl (manufactured by Wako Pure Chemical Industries, Ltd.) was added; 5 mL of a hexane solution (manufactured by Wako Pure Chemical Industries, Ltd.) containing 0.1% by mass of octanol (manufactured by Wako Pure Chemical Industries, Ltd.) was then added; and the contents were vigorously stirred to extract menthol into the hexane layer. The resultant was centrifuged with a centrifuge (manufactured by AS ONE Corporation, CN-810) under a condition at 5,000 rpm for 10 minutes, to separate the hexane layer from the water layer, and the hexane layer was then collected and further diluted by the addition of 10 mL of hexane.

The resulting solution was filtered with a PTFE filter having a mesh diameter of 0.45 µm, and the filtrate was measured with a gas chromatograph (manufactured by Agilent Technologies Japan, Ltd., 6850 Series II) (injection temperature: 220°C, carrier gas: helium, carrier gas flow rate: 1.2 mL/min, detector: FID, column: methyl silicone-based column (SE-30), column size = 30 m*250 µm*0.25 µm*). The menthol content in the sample was calculated from a peak area ratio of octanol as an internal standard to menthol. On the assumption that only the moisture was volatilized from the emulsion, when the amount of menthol contained in the powder was defined as a theoretical amount, a proportion of the menthol content in the sample to the theoretical amount was designated as the menthol residual rate (%).

### (iii) Release rates of menthol of salt-sensitive particles to 1% by mass and 10% by mass salt waters

About 0.03 g of the salt-sensitive particles containing menthol were weighed and preliminarily dispersed in 0.2 mL of propylene glycol (manufactured by Wako Pure Chemical Industries, Ltd.); 5 mL of 1% by mass salt water was then added; and the resultant was mixed in a 20-mL beaker at room temperature (25°C) for 5 minutes while stirring (50 rpm) with a stirrer having a size of 0.5 cm. Thereafter, the mixture was filtered with a PTFE filter having a mesh diameter of 1.2 µm, and 2.5 mL of DMSO (manufactured by Wako Pure Chemical Industries, Ltd.) was added.

To the aforementioned solution, 2 g of NaCl (manufactured by Wako Pure Chemical Industries, Ltd.) was added; 3 mL of a hexane solution (manufactured by Wako Pure Chemical Industries, Ltd.) containing 0.1% by mass of octanol (manufactured by Wako Pure Chemical Industries, Ltd.) was then added; and the contents were vigorously stirred to extract menthol into the hexane layer. The resultant was centrifuged with a centrifuge (manufactured by AS ONE Corporation, CN-810) under a condition at 5,000 rpm for 10 minutes, to separate the hexane layer from the water layer, and the hexane layer was then collected and further diluted by the addition of 10 mL of hexane.

The resulting solution was filtered with a PTFE filter having a mesh diameter of 0.45 µm, and the filtrate was measured with a gas chromatograph (manufactured by Agilent Technologies Japan, Ltd., 6850 Series II) (injection temperature: 220°C, carrier gas: helium, carrier gas flow rate: 1.2 mL/min, detector: FID, column: methyl silicone-based column (SE-30), column size = 30 m*250 µm*0.25 µm*). The amount of menthol eluted into the 1% by mass salt water was calculated, and a proportion of the amount of menthol eluted into the 1% by mass salt water to the amount of menthol contained in the powder as calculated by the analysis method (ii) was designated as a release rate (%) of menthol to the 1% by mass salt water.

In addition, with respect to a release rate (%) of menthol to the 10% by mass salt water, the analysis and calculation were similarly performed using the 10% by mass salt water according to the aforementioned method.

### (iv) Dispersion diameter of oil agent in salt-sensitive particles

Each particle was cleaved with a surgical knife (manufactured by Keisei Medical Industrial Co., Ltd.); a cross section of the particle was photographed (5,000 times) with a scanning electron microscope (manufactured by Keyence Corporation, VE-7800); and 50 pores (oil agent) existing in the cross section were chosen at random, and a maximum length (pore of 0.01 pm or more) thereof was measured. A number average value of the 50 pores was designated as an average dispersion diameter of the oil agent of the salt-sensitive particles. On the occasion of cleaving the particles described in the following Examples, in the case where the number of pores (derived from the oil agent) is less than 50, by increasing the number of cleavages, the number of pores can be regulated to 50. However, though particles obtained by the spray drying method are a hollow particle, the oil agent does not exist in the hollow pores, and therefore, the foregoing particles are not included in the measurement value. In addition, the oil agent is typically vaporized on the occasion of photographing (vacuum) with the scanning electron microscope.

### Example 1

So as to have a blending proportion shown in Table 1, 7,239.4 g of ion-exchanged water was subjected to temperature rise to 80°C, and 493.6 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

248.4 g of menthol (manufactured by Takasago International Corporation, Menthol JP(TAB)COS), 9.3 g of polyoxyethylene (20EO) sorbitan monostearate (manufactured by Kao Corporation, RHEODOL TW-S120V), and 9.3 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 23.5% of menthol. 10 or more pores derived from the oil agent existed on the cleaved surface of the salt-sensitive particle obtained in Example 1. The same was applied in the following Examples 2 to 16.

### Example 2

So as to have a blending proportion shown in Table 1, 7,202.3 g of ion-exchanged water was subjected to temperature rise to 80°C, and 493.6 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

248.4 g of menthol (manufactured by Takasago International Corporation, Menthol JP(TAB)COS), 27.9 g of polyoxyethylene (20EO) sorbitan monostearate (manufactured by Kao Corporation, RHEODOL TW-S120V), and 27.9 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a propeller stirring blade having a diameter of 120 mm at 100 r/min for 30 minutes, to prepare an emulsion (50°C). Thereafter, the emulsion was subjected to a high-pressure emulsification treatment with a high-pressure emulsifier (nanomizer, 130 MPa, one pass).

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 27.3% of menthol.

### Example 3

So as to have a blending proportion shown in Table 1, 7,176 g of ion-exchanged water was subjected to temperature rise to 80°C, and 496 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

248 g of menthol (manufactured by Takasago International Corporation, Menthol JP(TAB)COS), 40 g of polyoxyethylene (20EO) sorbitan monostearate (manufactured by Kao Corporation, RHEODOL TW-S120V), and 40 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a propeller stirring blade having a diameter of 120 mm at 100 r/min for 30 minutes, to prepare an emulsion (50°C).

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.3% (measured at 105°C with a moisture meter MOC63u, manufactured by Shimadzu Corporation). As a result of gas chromatographic analysis, the powder contained 22.8% of menthol.

### Example 4

So as to have a blending proportion shown in Table 1, 7,258.3 g of ion-exchanged water was subjected to temperature rise to 80°C, and 493.4 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

248.3 g of menthol (manufactured by Takasago International Corporation, Menthol JP(TAB)COS) was subjected to temperature rise to 50°C, and the resultant was melted to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 2.0% (measured at 105°C with a moisture meter MOC63u, manufactured by Shimadzu Corporation). As a result of gas chromatographic analysis, the powder contained 17.1% of menthol.

### Example 5

So as to have a blending proportion shown in Table 1, 7,256 g of ion-exchanged water was subjected to temperature rise to 80°C, and 496 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

248 g of menthol (manufactured by Takasago International Corporation, Menthol JP(TAB)COS) was subjected to temperature rise to 50°C, and the resultant was melted to prepare an oil phase.

The oil phase was added to the prepared water phase, and the contents were subjected to a stirring operation with a propeller stirring blade having a diameter of 120 mm at 100 r/min for 30 minutes, to prepare an emulsion (50°C).

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 2.0% (measured at 105°C with a moisture meter MOC63u, manufactured by Shimadzu Corporation). As a result of gas chromatographic analysis, the powder contained 15.1% of menthol.

### Example 6

So as to have a blending proportion shown in Table 1, 3,462.5 g of ion-exchanged water was subjected to temperature rise to 80°C, and 1,034 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and melted/dispersed, followed by cooling to 50°C to prepare a water phase.

468.5 g of menthol (manufactured by Takasago International Corporation, Menthol JP(TAB)COS), 15.0 g of polyoxyethylene (20EO) sorbitan monostearate (manufactured by Kao Corporation, RHEODOL TW-S120V), and 15.0 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a propeller stirring blade having a diameter of 120 mm at 100 r/min for 30 minutes, to prepare an emulsion (50°C).

The emulsion obtained by the aforementioned emulsification operation was dried with a drum dryer (manufactured by Katsuragi Industry Co., Ltd., ϕ400 mm single drum dryer) under a condition at an emulsion feed rate of 1,800 g/hr, at a feed vapor pressure of 0.12 MPa (drum surface temperature: 120°C), and a drum rotation number of 0.3 rpm, to obtain a sheet-shaped dried product. The sheet was crushed into chips with an office shredder (manufactured by ACCO BRANDS Japan K.K.) and then crushed into a powder using a pin mill crusher (manufactured by Hosokawa Micron Corporation, Fine Impact Mill) to form a powder having an average particle diameter of 670 pm.

A loss on drying of the prepared powder was 4.9% (measured at 105°C with a moisture meter MOC63u, manufactured by Shimadzu Corporation). As a result of gas chromatographic analysis, the powder contained 21.7% of menthol.

### Example 7

So as to have a blending proportion shown in Table 2, 7,239.4 g of ion-exchanged water was subjected to temperature rise to 80°C, and 493.6 g of an acid-modified polyvinyl alcohol (GOHSENX T-330H, manufactured by The Nippon Synthetic Chemical Industry Co., Ltd., degree of polymerization = 2,000, degree of saponification = 95 to 98 mol%, modified with carboxylic acid) was then added and dissolved, followed by cooling to 50°C to prepare a water phase.

248.4 g of menthol (manufactured by Takasago International Corporation, Menthol JP(TAB)COS), 9.3 g of polyoxyethylene (20EO) sorbitan monostearate (manufactured by Kao Corporation, RHEODOL TW-S120V), and 9.3 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 26.1% of menthol.

### Example 8

So as to have a blending proportion shown in Table 2, 7,239.4 g of ion-exchanged water was subjected to temperature rise to 80°C, and 493.6 g of an acid-modified polyvinyl alcohol (GOHSENX T-350, manufactured by The Nippon Synthetic Chemical Industry Co., Ltd., degree of polymerization = 2,000, degree of saponification = 93 to 95 mol%, modified with carboxylic acid) was then added and dissolved, followed by cooling to 50°C to prepare a water phase.

248.4 g of menthol (manufactured by Takasago International Corporation, Menthol JP(TAB)COS), 9.3 g of polyoxyethylene (20EO) sorbitan monostearate (manufactured by Kao Corporation, RHEODOL TW-S120V), and 9.3 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 25.4% of menthol.

### Example 9

So as to have a blending proportion shown in Table 2, 7,143.9 g of ion-exchanged water was subjected to temperature rise to 80°C, and 493.6 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

337.2 g of menthol (manufactured by Takasago International Corporation, Menthol JP(TAB)COS), 12.6 g of polyoxyethylene (20EO) sorbitan monostearate (manufactured by Kao Corporation, RHEODOL TW-S120V), and 12.6 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 27.2% of menthol.

### Example 10

So as to have a blending proportion shown in Table 2, 6,899.2 g of ion-exchanged water was subjected to temperature rise to 80°C, and 493.6 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

335.3 g of menthol (manufactured by Takasago International Corporation, Menthol JP(TAB)COS), 12.6 g of polyoxyethylene (20EO) sorbitan monostearate (manufactured by Kao Corporation, RHEODOL TW-S120V), and 12.6 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C). Furthermore, 246.8 g of corn starch (manufactured by Matsutani Chemical Industry Co., Ltd., corn starch of Japanese Pharmacopoeia, JP) was added to the emulsion, and the contents were subjected to a stirring operation with a disper stirring blade, manufactured by PRIMIX Corporation at 3,000 r/min for 30 minutes, to prepare an emulsion (50°C).

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 21.3% of menthol.

### Example 11

So as to have a blending proportion shown in Table 3, 7,240 g of ion-exchanged water was subjected to temperature rise to 80°C, and 496 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

232 g of dipentaerythrityl tripolyhydroxystearate (manufactured by The Nisshin OilliO Group, Ltd., SALACOS WO-6), 16 g of polyoxyethylene (20EO) sorbitan monostearate (nonionic surfactant, manufactured by Kao Corporation, RHEODOL TW-S120V), and 16 g of polyoxyethylene (6EO) tridecyl ether sodium acetate (anionic surfactant, manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a moisturizer-containing powder. A loss on drying of the prepared powder was 1.5% (measured at 105°C with a moisture meter MOC63u, manufactured by Shimadzu Corporation).

### Example 12

So as to have a blending proportion shown in Table 3, 7,248 g of ion-exchanged water was subjected to temperature rise to 80°C, and 496 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was then added and dissolved, followed by cooling to 50°C to prepare a water phase.

112 g of dipentaerythrityl tripolyhydroxystearate (manufactured by The Nisshin OilliO Group, Ltd., SALACOS WO-6), 112 g of vaseline (manufactured by SONNEBORN, LLC, Superwhite Protopet), 16 g of polyoxyethylene (20EO) sorbitan monostearate (nonionic surfactant, manufactured by Kao Corporation, RHEODOL TW-S120V), and 16 g of polyoxyethylene (6EO) tridecyl ether sodium acetate (anionic surfactant, manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a moisturizer-containing powder. A loss on drying of the prepared powder was 1.5% (measured at 105°C with a moisture meter MOC63u, manufactured by Shimadzu Corporation).

### Example 13

So as to have a blending proportion shown in Table 4, 14.01 kg of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was added to and dispersed in 76.59 kg of ion-exchanged water, and the dispersion was then subjected to temperature rise to 80°C for dissolution. Thereafter, the resulting solution was cooled to 75°C to prepare a water phase.

1.71 kg of dipentaerythrityl tripolyhydroxystearate (manufactured by The Nisshin OilliO Group, Ltd., SALACOS WO-6), 1.71 kg of vaseline (manufactured by SONNEBORN, LLC, Superwhite Protopet), 3,43 kg of squalane (manufactured by Nippon Surfactant Industries Co., Ltd., NIKKOL Squalane), 1.54 kg g of polyoxyethylene (20EO) sorbitan monostearate (nonionic surfactant, manufactured by Kao Corporation, RHEODOL TW-S120V), and 0.51 kg of sorbitan stearate (nonionic surfactant, manufactured by Kao Corporation, RHEODOL SP-S10V) were added and then subjected to temperature rise to 75°C, and the resultant was melted/dispersed to prepare an oil phase.

The oil phase was added to the prepared water phase, and the contents were subjected to a stirring operation with an anchor stirring blade having a diameter of 505 mm at 49 r/min for 30 minutes (75°C). Thereafter, 200.49 kg of ion-exchanged water was added to prepare an emulsion having an average particle diameter of an emulsion diameter of 2.7 µm.

The emulsion obtained by the aforementioned emulsification operation was spray-dried with a spray dryer (manufactured by Niro Atomizer K.K., SPRAY DRYER) under a condition at an emulsion feed rate of 44 kg/hr, at a blowing temperature of 155°C, and at an exhausting temperature of 85°C, to obtain a moisturizer-containing powder. A loss on drying of the prepared powder was 1.5% (measured at 105°C with a moisture meter MOC63u, manufactured by Shimadzu Corporation). A yield of the obtained dried powder was 42%. The yield is a value calculated by [(amount of obtained particle solid component)/(amount of charged solid component) × 100].

### Example 14

So as to have a blending proportion shown in Table 4, 14.01 kg of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was added to and dispersed in 76.59 kg of ion-exchanged water, and the dispersion was then subjected to temperature rise to 80°C for dissolution. Thereafter, the resulting solution was cooled to 75°C to prepare a water phase.

1.71 kg of dipentaerythrityl tripolyhydroxystearate (manufactured by The Nisshin OilliO Group, Ltd., SALACOS WO-6), 1.71 kg of vaseline (manufactured by SONNEBORN, LLC, Superwhite Protopet), 3.43 kg of squalane (manufactured by Nippon Surfactant Industries Co., Ltd., NIKKOL Squalane), 1.54 kg g of polyoxyethylene (20EO) sorbitan monostearate (nonionic surfactant, manufactured by Kao Corporation, RHEODOL TW-S120V), and 0.51 kg of sorbitan stearate (nonionic surfactant, manufactured by Kao Corporation, RHEODOL SP-S10V) were added and then subjected to temperature rise to 75°C, and the resultant was melted/dispersed to prepare an oil phase.

The oil phase was added to the prepared water phase, and the contents were stirred with an anchor stirring blade having a diameter of 505 mm at 94 r/min for 82 minutes. At the same time, while circulating the mixed phase at a rate of 120 L/hr, the circulated liquid was subjected to a dispersion operation with a milder (manufactured by Pacific Machinery & Engineering Co., Ltd., MDN-303V) at 10,000 r/min. Thereafter, 200.49 kg of ion-exchanged water was added to prepare an emulsion having an average particle diameter of an emulsion diameter of 0.4 pm.

The emulsion obtained by the aforementioned emulsification operation was spray-dried with a spray dryer (manufactured by Niro Atomizer K.K., SPRAY DRYER) under a condition at an emulsion feed rate of 44 kg/hr, at a blowing temperature of 155°C, and at an exhausting temperature of 85°C, to obtain a moisturizer-containing powder. A loss on drying of the prepared powder was 1.5% (measured at 105°C with a moisture meter MOC63u, manufactured by Shimadzu Corporation). A yield of the obtained dried powder was 51%.

### Example 15

So as to have a blending proportion shown in Table 4, 14.01 kg of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was added to and dispersed in 76.59 kg of ion-exchanged water, and the dispersion was then subjected to temperature rise to 80°C for dissolution. Thereafter, the resulting solution was cooled to 75°C to prepare a water phase.

2.58 kg of dipentaerythrityl tripolyhydroxystearate (manufactured by The Nisshin OilliO Group, Ltd., SALACOS WO-6), 2.58 kg of vaseline (manufactured by SONNEBORN, LLC, Superwhite Protopet), 5.16 kg of squalane (manufactured by Nippon Surfactant Industries Co., Ltd., NIKKOL Squalane), 2.32 kg g of polyoxyethylene (20EO) sorbitan monostearate (nonionic surfactant, manufactured by Kao Corporation, RHEODOL TW-S120V), and 0.77 kg of sorbitan stearate (nonionic surfactant, manufactured by Kao Corporation, RHEODOL SP-S10V) were added and then subjected to temperature rise to 75°C, and the resultant was melted/dispersed to prepare an oil phase.

The oil phase was added to the prepared water phase, and the contents were stirred with an anchor stirring blade having a diameter of 505 mm at 94 r/min for 40 minutes. At the same time, while circulating the mixed phase at a rate of 1,700 L/hr, the circulated liquid was subjected to a dispersion operation with a milder (manufactured by Pacific Machinery & Engineering Co., Ltd., MDN-307) at 8,000 r/min. Thereafter, 189.10 kg of ion-exchanged water, 3.44 kg of trehalose (manufactured by Hayashibara Co., Ltd.), and 3.44 kg of dextrin (H-PDx, manufactured by Matsutani Chemical Industry Co., Ltd.) were added to prepare an emulsion having an average particle diameter of an emulsion diameter of 0.4 pm.

The emulsion obtained by the aforementioned emulsification operation was spray-dried with a spray dryer (manufactured by Niro Atomizer K.K., SPRAY DRYER) under a condition at an emulsion feed rate of 44 kg/hr, at a blowing temperature of 155°C, and at an exhausting temperature of 85°C, to obtain a moisturizer-containing powder. A loss on drying of the prepared powder was 1.5% (measured at 105°C with a moisture meter MOC63u, manufactured by Shimadzu Corporation). A yield of the obtained dried powder was 50%.

### Example 16

After subjecting 878.3 g of ion-exchanged water to temperature rise to 80°C, 150 g of an acid-modified polyvinyl alcohol (GOHSENX T-330H, manufactured by The Nippon Synthetic Chemical Industry Co., Ltd., degree of polymerization = 2,000, degree of saponification > 99 mol%, modified with carboxylic acid) was added and dispersed/dissolved, followed by cooling to 50°C to paper a water phase.

75.5 g of menthol (manufactured by Takasago International Corporation, Menthol JP(TAB)COS), 2.8 g of polyoxyethylene (20EO) sorbitan monostearate (nonionic surfactant, manufactured by Kao Corporation, RHEODOL TW-S 120V), and 2.8 g of polyoxyethylene (6EO) tridecyl ether sodium acetate (anionic surfactant, manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a propeller stirring blade having a diameter of 120 mm at 100 r/min for 30 minutes, to prepare an emulsion having an average particle diameter of an emulsion diameter of 0.3 pm (50°C).

So as to have a blending proportion shown in Table 5, 54 g of the emulsion obtained by the aforementioned emulsification operation and 100 g of a cellulose powder (manufactured by Nippon Paper Industries, Co., Ltd., KC FLOCK W-400G, average particle diameter = 24 pm) were subjected to stirring granulation with a food mixer (manufactured by Yamamoto Electric Corporation, MM41) at a stirring rotation number of 1,900 rpm for 1 minute. The obtained granulated product was dried with an electric dryer (manufactured by Advantec Co., Ltd., DRM620TB) at a hot air temperature of 100°C for 1 hour, to obtain a granulated product. A loss on drying of the prepared granulated product was 4.3% (measured at 105°C with a moisture meter MOC63u, manufactured by Shimadzu Corporation). As a result of gas chromatographic analysis, the powder contained 1.1% of menthol.

### Comparative Example 1

So as to have a blending proportion shown in Table 2, 496 g of a vinyl acetate/vinylpyrrolidone copolymer (Luviskol VA64P, manufactured by BASF SE) was added to and dissolved in 7,176 g of ion-exchanged water, and the resultant was then subjected to temperature rise to 50°C to prepare a water phase.

248 g of menthol (manufactured by Takasago International Corporation, Menthol JP(TAB)COS), 40 g of polyoxyethylene (20EO) sorbitan monostearate (manufactured by Kao Corporation, RHEODOL TW-S120V), and 40 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 4.2% of menthol.

### Comparative Example 2

So as to have a blending proportion shown in Table 2, 7,176 g of ion-exchanged water was subjected to temperature rise to 80°C, and 496 g of unmodified PVA (PVA-117, manufactured by Kuraray Co., Ltd., degree of polymerization = 1,800, degree of saponification: 99 mol%) was added and dispersed/dissolved, followed by cooling to 50°C to prepare a water phase.

248 g of menthol (manufactured by Takasago International Corporation, Menthol JP(TAB)COS), 40 g of polyoxyethylene (20EO) sorbitan monostearate (manufactured by Kao Corporation, RHEODOL TW-S120V), and 40 g of polyoxyethylene (6EO) tridecyl ether sodium acetate (manufactured by Nikko Chemicals Co., Ltd., ECTD-6NEX) were added and then subjected to temperature rise to 50°C, and the resultant was melted/dispersed to prepare an oil phase.

The oil phase was added to the prepared water phase, and the contents were subjected to a stirring operation with a disper stirring blade, manufactured by PRIMIX Corporation at 3,000 r/min for 30 minutes, to prepare an emulsion (50°C) .

The emulsion obtained by the aforementioned emulsification operation was spray-dried with a spray dryer (manufactured by Sakamoto Giken Co., Ltd., SPRAY DRYER) under a condition at an emulsion feed rate of 4,900 g/hr, at a blowing temperature of 150°C, and at an exhausting temperature of 90°C, to obtain a menthol-containing powder. A loss on drying of the prepared powder was 1.5% (measured at 105°C with a moisture meter MOC63u, manufactured by Shimadzu Corporation). As a result of gas chromatographic analysis, the powder contained 22.8% of menthol.

Organoleptic evaluations were performed by the following methods.

### Evaluation of Example 1

Skin cleaning compositions expressed in Formulation 1 in which the particles obtained in Example 1 were blended in an amount of 2% such that the amount of menthol in the formulation was 0.47%, and in Formulation 2 in which menthol was directly blended in an amount of 0.47% in the formulation were produced by the following conventional method, and the following evaluation (1) was performed. The results are shown in Table 1.

After facial cleaning by Formulation 1, a strong feeling of coolness was obtained on the cheek and forehead, whereas a burning sensation was not felt so much in the eyes.

### Evaluation of Examples 2 to 10 and Comparative Examples 1 to 2

By regulating the blending amount of the particles in place of the particles of Example 1, skin cleaning compositions expressed in Formulation 1 in which the particles obtained in each of the Examples and Comparative Examples were blended such that the amount of menthol in the formulation was 0.47%, and shown in Formulation 2 in which menthol was directly blended in an amount of 0.47% in the formulation were produced by the following conventional method, and the following evaluation (1) was performed. The results are shown in Tables 1 and 2.

After facial cleaning in each of Examples 2 to 10, similar to the case after facial cleaning in Example 1, a strong feeling of coolness was obtained on the cheek and forehead, whereas a burning sensation was not felt so much in the eyes.

### Evaluation of Example 11

Skin cleaning compositions expressed in Formulation 1 in which 1.7% of the particles obtained in Example 11 was blended in place of the particles of Example 1 such that the amount of the moisturizer (dipentaerythrityl tripolyhydroxystearate) in the formulation was 0.5%, and in Formulation 2 in which dipentaerythrityl tripolyhydroxystearate was directly blended in an amount of 0.5% in place of 0.47% of the amount of menthol were produced by the following conventional method, and the following evaluations (2) to (3) and (5) were performed. The results are shown in Table 3.

### Evaluation of Example 12

Skin cleaning compositions expressed in Formulation 1 in which 1.7% of the particles obtained in Example 11 was blended in place of the particles of Example 1 such that the amount of the moisturizer (dipentaerythrityl tripolyhydroxystearate and vaseline) in the formulation was 0.5%, and in Formulation 2 in which dipentaerythrityl tripolyhydroxystearate in an amount of 0.25% and vaseline in an amount of 0.25% were directly blended in place of 0.47% of the amount of menthol were produced by the following conventional method, and the following evaluations (2) to (3) and (5) were performed. The results are shown in Table 3.

### Evaluation of Examples 13 to 15

Skin cleaning compositions expressed in Formulation 1 in which 2.0% of the particles obtained in each of Examples 13 to 15 was blended in place of the particles of Example 1 such that the amount of the moisturizer (dipentaerythrityl tripolyhydroxystearate, vaseline, and squalane) in the formulation was 0.6%, and in Formulation 2 in which dipentaerythrityl tripolyhydroxystearate in an amount of 0.15%, vaseline in an amount of 0.15%, and squalane in an amount of 0.3% were directly blended in place of 0.47% of the amount of menthol were produced by the following conventional method, and the following evaluations (2) to (3) and (5) were performed. The results are shown in Table 4.

In the direct blending in Formulation 2, after storing the preparation at 50°C for one month, oil floating was found, whereas in Formulation 1 in which the salt-sensitive particles were blended, no oil floating was found.

### Evaluation of Example 16

Skin cleaning compositions expressed in Formulation 1 in which 9.1% of the particles obtained in Example 16 was blended in place of the particles of Example 1 such that the amount of menthol in the formulation was 0.1%, and in Formulation 2 in which menthol in an amount of 0.1% was directly blended in place of 0.47% of the amount of menthol were produced by the following conventional method, and the following evaluation (1) was performed. The results are shown in Table 5.

The formulation having the particles of Example 16 blended therein was also excellent in grain feeling (scrubbing feeling) at the time of facial cleaning.

### Evaluation of Examples 17 to 19

Skin cleaning compositions expressed in Formulation 3 in which the particles obtained in each of Examples 7 to 9 were blended such that the amount of menthol in the formulation was 0.47%, and in Formulation 4 in which menthol was directly blended such that its amount in the formulation was 0.47% were produced by the following conventional method, and the following evaluation (4) was performed.

In all of the Examples, the strength of feeling of coolness was 4.0, and a strong feeling of coolness was obtained on the cheek and forehead, whereas a burning sensation was not felt so much in the eyes.

### Evaluation of Examples 20 to 22

Skin cleaning compositions expressed in Formulation 5 in which the particles obtained in each of Examples 7 to 9 were blended such that the amount of menthol in the formulation was 0.47%, and in Formulation 6 in which menthol was directly blended such that its amount in the formulation was 0.47% were produced by the following conventional method, and the following evaluation (4) was performed.

In all of the Examples, the strength of feeling of coolness was 4.0, and a strong feeling of coolness was obtained on the cheek and forehead, whereas a burning sensation was not felt so much in the eyes.

### (1) Feeling of Coolness

For four expert panelists, 2 g of each of the skin cleaning compositions was diluted with water in a small amount of 4 g and applied for cleaning so as to massage a face with fixed speed and force for 30 seconds, and the feeling of coolness on the cheek and forehead after facial cleaning was evaluated according to the following criteria.
5: The feeling of coolness utterly stronger than that in Formulation 2 is provided.
4: The feeling of coolness stronger than that in Formulation 2 is provided.
3: The feeling of coolness equal to that in Formulation 2 is provided.
2: The feeling of coolness is not provided as compared with that in Formulation 2.
1: The feeling of coolness is not provided at all as compared with that in Formulation 2.

### (2) Moist Feeling

For four expert panelists, 2 g of each of the skin cleaning compositions was diluted with water in a small amount of 4 g and applied for cleaning so as to massage a face with fixed speed and force for 30 seconds, and the moist feeling after facial cleaning was evaluated according to the following criteria.
5: The moist feeling utterly stronger than that in Formulation 2 is provided.
4: The moist feeling stronger than that in Formulation 2 is provided.
3: The moist feeling equal to that in Formulation 2 is provided.
2: The moist feeling is not provided as compared with that in Formulation 2.
1: The moist feeling is not provided at all as compared with that in Formulation 2.

### (3) Stretched Feeling

For four expert panelists, 2 g of each of the skin cleaning compositions was diluted with water in a small amount of 4 g and applied for cleaning so as to massage a face with fixed speed and force for 30 seconds, and the stretched feeling after facial cleaning was evaluated according to the following criteria.
5: The stretched feeling is not provided at all as compared with that in Formulation 2.
4: The stretched feeling is not provided as that in Formulation 2 is provided.
3: The stretched feeling equal to that in Formulation 2 is provided.
2: The stretched feeling stronger than that in Formulation 2 is provided.
1: The stretched feeling utterly stronger than that in Formulation 2 is provided.

### (4) Feeling of Coolness

For four expert panelists, 2 g of each of the skin cleaning compositions was diluted with water in a small amount of 4 g and applied for cleaning so as to massage a face with fixed speed and force for 30 seconds, and the feeling of coolness on the cheek and forehead after facial cleaning was evaluated according to the following criteria.
5: The feeling of coolness utterly stronger than that in Formulation 4 is provided.
4: The feeling of coolness stronger than that in Formulation 4 is provided.
3: The feeling of coolness equal to that in Formulation 4 is provided.
2: The feeling of coolness is not provided as compared with that in Formulation 4.
1: The feeling of coolness is not provided at all as compared with that in Formulation 4.

### (5) Presence or Absence of Oil Floating

50 g of each of the skin cleaning compositions obtained by the production method of Formulation 1 or Formulation 2 was hermetically sealed in a glass container (manufactured by Maruemu Corporation, No. 7, capacity: 50 mL) and stored at 50°C for one month. Thereafter, the presence or absence of oil floating was visually confirmed and evaluated according to the following criteria.
2: The oil floating is not found as compared with that in Formulation 2, and the storage stability is good.
1: The oil floating is found similar to that in Formulation 2, and the storage stability is poor.

**Table 1**

| | | Example 1 | | Example 2 | | Example 3 | | Example 4 | | Example 5 | | Example 6 | |
|---|---|---|---|---|---|---|---|---|---|---|---|---|---|
| | | Emulsification composition | Dried product | Emulsification composition | Dried product | Emulsification composition | Dried product | Emulsification composition | Dried product | Emulsification composition | Dried product | Emulsification composition | Dried product |
| | | mass% | mass% | mass% | mass% | mass% | mass% | mass% | mass% | mass% | mass% | mass% | mass% |
| Formulation | Acid-modified polyvinyl alcohol-1 | 6.2 | 72.3 | 6.2 | 64.0 | 6.2 | 65.9 | 6.2 | 80.9 | 6.2 | 82.9 | 20.7 | 71.3 |
| | Acid-modified polyvinyl alcohol-2 | | | | | | | | | | | | |
| | Acid-modified polyvinyl alcohol-3 | | | | | | | | | | | | |
| | Vinyl acetate/ vinylpyrrolidone copolymer | | | | | | | | | | | | |
| | Unmodified polyvinyl alcohol | | | | | | | | | | | | |
| | Menthol | 3.1 | 23.5 | 3.1 | 27.3 | 3.1 | 22.8 | 3.1 | 17.1 | 3.1 | 15.1 | 9.4 | 21.7 |
| | Nonionic surfactant-1 | 0.1 | 1.4 | 0.3 | 3.6 | 0.5 | 5.0 | | | | | 0.3 | 1.0 |
| | Anionic surfactant | 0.1 | 1.4 | 0.3 | 3.6 | 0.5 | 5.0 | | | | | 0.3 | 1.0 |
| | Corn starch | | | | | | | | | | | | |
| | Ion-exchanged water | 90.5 | 1.5 | 90.0 | 1.5 | 89.7 | 1.3 | 90.7 | 2.0 | 90.7 | 2.0 | 69.3 | 4.9 |
| | Total | 100 | 100 | 100 | 100 | 100 | 100 | 100 | 100 | 100 | 100 | 100 | 100 |
| | Acid-modified polyvinyl alcohol/Oil agent | | 3.1 | | 2.3 | | 2.9 | | 4.7 | | 5.5 | | 3.3 |
| Emulsification | Emulsification method | Disper emulsification at 3,000 rpm | | High -pressure emulsification at 130 MPa | | Propeller emulsification at 100 rpm | | Disper emulsification at 3,000 rpm | | Propeller emulsification at 100 rpm | | Propeller emulsification at 100 rpm | |
| | Emulsion average particle diameter [µm] | 0.40 | | 0.16 | | 4.8 | | 37.8 | | 62.0 | | 0.40 | |
| Properties of dried product | Drying method | Spray drying at 150°C | | Spray drying at 150°C | | Spray drying at 150°C | | Spray drying at 150°C | | Spray drying at 150°C | | Drum dryer Vapor pressure: 0.12 MPa | |
| | Powder average particle diameter of salt-sensitive particles [µm] | 79 | | 62 | | 65 | | 58 | | 48 | | 670 | |
| | Residual rate of menthol [%] | 73 | | 89 | | 76 | | 52 | | 46 | | 75 | |
| | Release rate of oil agent in 10% salt water S₁₀ [%] | 8 | | - | | 13 | | 12 | | 13 | | - | |
| | Release rate of oil agent in 1% salt water S₁ [%] | 44 | | - | | 40 | | 42 | | 41 | | - | |
| | S₁ - S₁₀ [%] | 36 | | - | | 27 | | 30 | | 28 | | - | |
| | Average dispersion diameter of oil agent [µm] | 0.45 | | 0.21 | | 1.28 | | 3.12 | | 4.00 | | 1.20 | |
| | (Average dispersion diameter of oil agent)/ (Average particle diameter of salt-sensitive particles) | 0.006 | | 0.003 | | 0.020 | | 0.054 | | 0.083 | | 0.002 | |
| Evaluation | Strength of feeling of coolness (menthol amount: 0.47%) | 4.5 | | 5.0 | | 4.0 | | 3.5 | | 3.5 | | 4.0 | |

**Table 2**

| | | Example 7 | | Example 8 | | Example 9 | | Example 10 | | Comparative Example 1 | | Comparative Example 2 | |
|---|---|---|---|---|---|---|---|---|---|---|---|---|---|
| | | Emulsification composition | Dried product | Emulsification composition | Dried product | Emulsification composition | Dried product | Emulsification composition | Dried product | Emulsification composition | Dried product | Emulsification composition | Dried product |
| | | mass% | mass% | mass% | mass% | mass% | mass% | mass% | mass% | mass% | mass% | mass% | mass% |
| Formulation | Acid-modified polyvinyl alcohol-1 | | | | | 6.2 | 67.9 | 6.2 | 49.8 | | | | |
| | Acid-modified polyvinyl alcohol-2 | 6.2 | 69.8 | | | | | | | | | | |
| | Acid-modified polyvinyl alcohol-3 | | | 6.2 | 70.4 | | | | | | | | |
| | Vinyl acetate/ vinylpyrrolidone copolymer | | | | | | | | | 6.2 | 81.9 | | |
| | Unmodified polyvinyl alcohol | | | | | | | | | | | 6.2 | 65.8 |
| | Menthol | 3.1 | 26.1 | 3.1 | 25.4 | 4.2 | 27.2 | 4.2 | 21.3 | 3.1 | 4.2 | 3.1 | 22.8 |
| | Nonionic surfactant-1 | 0.1 | 1.3 | 0.1 | 1.3 | 0.2 | 1.7 | 0.2 | 1.3 | 0.5 | 6.2 | 0.5 | 5.0 |
| | Anionic surfactant | 0.1 | 1.3 | 0.1 | 1.3 | 0.2 | 1.7 | 0.2 | 1.3 | 0.5 | 6.2 | 0.5 | 5.0 |
| | Corn starch | | | | | | | 3.1 | 24.9 | | | | |
| | Ion-exchanged water | 90.5 | 1.5 | 90.5 | 1.5 | 89.3 | 1.5 | 86.2 | 1.5 | 89.7 | 1.5 | 89.7 | 1.5 |
| | Total | 100 | 100 | 100 | 100 | 100 | 100 | 100 | 100 | 100 | 100 | 100 | 100 |
| | Acid-modified polyvinyl alcohol/Oil agent | | 27 | | 2.8 | | 2.5 | | 2.3 | | | | |
| Emulsification | Emulsification method | Disper emulsification at 3,000 rpm | | Disper emulsification at 3,000 rpm | | Disper emulsification at 3,000 rpm | | Disper emulsification at 3,000 rpm | | Disper emulsification at 3,000 rpm | | Disper emulsification at 3,000 rpm | |
| | Emulsion average particle diameter [µm] | 0.66 | | 0.46 | | 0.78 | | 0.56 | | 3.4 | | 0.46 | |
| Properties of dried product | Drying method | Spray drying at 150°C | | Spray drying at 150°C | | Spray drying at 150°C | | Spray drying at 150°C | | Spray drying at 150°C | | Spray drying at 150°C | |
| | Powder average particle diameter of salt-sensitive particles [µm] | 57 | | 63 | | 67 | | 57 | | 15 | | 52 | |
| | Residual rate of menthol [%] | 81 | | 79 | | 70 | | 71 | | 14 | | 76 | |
| | Release rate of oil agent in 10% salt water S₁₀ [%] | 10 | | 9 | | - | | - | | 85 | | 8 | |
| | Release rate of oil agent in 1% salt water S₁ [%] | 49 | | 59 | | - | | - | | 90 | | 18 | |
| | S₁ - S₁₀ [%] | 39 | | 50 | | - | | - | | 5 | | 10 | |
| | Average dispersion diameter of oil agent [µm] | 0.54 | | 0.69 | | - | | - | | - | | 0.61 | |
| | (Average dispersion diameter of oil agent)/ (Average particle diameter of salt-sensitive particles) | 0.009 | | 0.011 | | - | | - | | - | | 0.012 | |
| Evaluation | Strength of feeling of coolness (menthol amount: 0.47%) | 4.5 | | 4.5 | | 4.5 | | 4.5 | | 3.0 | | 1.0 | |

**Table 3**

| | | Example 11 | | Example 12 | |
|---|---|---|---|---|---|
| | | Emulsification composition | Dried product | Emulsification composition | Dried product |
| | | mass% | mass% | mass% | mass% |
| Formulation | Acid-modified polyvinyl alcohol-1 | 6.2 | 64.3 | 6.2 | 65.0 |
| | Dipentaerythrityl tripolyhydroxystearate | 2.9 | 30.1 | 1.4 | 14.7 |
| | Vaseline | | | 1.4 | 14.7 |
| | Nonionic surfactant-1 | 0.2 | 2.1 | 0.2 | 2.1 |
| | Anionic surfactant | 0.2 | 2.1 | 0.2 | 2.1 |
| | Ion-exchanged water | 90.5 | 1.5 | 90.6 | 1.5 |
| | Total | 100 | 100 | 100 | 100 |
| | Acid-modified PVA/ Oil agent | | 2.1 | | 2.2 |
| Emulsification | Emulsification method | Disper emulsification at 3,000 rpm | | Disper emulsification at 3,000 rpm | |
| | Emulsion average particle diameter [µm] | 19 | | 11 | |
| Properties of dried product | Drying method | Spray drying at 150°C | | Spray drying at 150°C | |
| | Powder average particle diameter [µm] | 90 | | 72 | |
| | Average particle diameter of oil agent [µm] | 14.4 | | 8.1 | |
| | (Average dispersion diameter of oil agent)/ (Average particle diameter of salt-sensitive particles) | 0.160 | | 0.113 | |
| Evaluation | Presence or absence of oil floating | 2 | | 2 | |
| | Moist feel | 4.0 | | 4.5 | |
| | Nothing of stretched feeling | 4.0 | | 4.5 | |

**Table 4**

| | | Example 12 | | Example 14 | | Example 15 | |
|---|---|---|---|---|---|---|---|
| | | Emulsification composition | Dried product | Emulsification composition | Dried product | Emulsification composition | Dried product |
| | | mass% | mass% | mass% | mass% | mass% | mass% |
| Formulation | Acid-modified polyvinyl alchol-1 | 4.5 | 59.5 | 4.5 | 59.5 | 4.5 | 39.5 |
| | Dipentaerythrityl tripolyhydroxystearate | 0.6 | 7.5 | 0.6 | 7.5 | 0.9 | 7.5 |
| | Vaseline | 0.6 | 7.5 | 0.6 | 7.5 | 0.9 | 7.5 |
| | Squalane | 1.1 | 15.0 | 1.1 | 15.0 | 1.7 | 15.0 |
| | Nonionic surfactant-1 | 0.5 | 6.8 | 0.5 | 6.8 | 0.8 | 6.7 |
| | Nonionic surfactant-2 | 0.2 | 2.3 | 0.2 | 2.3 | 0.3 | 2.2 |
| | Trehalose | | | | | 1.1 | 10.0 |
| | Dextrin | | | | | 1.1 | 10.0 |
| | Ion-exchanged water | 92.5 | 1.5 | 92.5 | 1.5 | 88.7 | 1.5 |
| | Total | 100 | 100 | 100 | 100 | 100 | 100 |
| | Acid-modified PVA/Oil agent | | 2.0 | | 2.0 | | 1.3 |
| Emulsification | Emulsification method | Anchor blade emulsification at 49 rpm | | Anchor blade emulsification at 49 rpm Milder emulsification at 10,000 rpm | | Anchor blade emulsification at 49 rpm Milder emulsification at 10,000 rpm | |
| | Emulsion average particle diameter [µm] | 2.7 | | 0.4 | | 0.4 | |
| Properties of dried product | Drying method | Spray drying at 155°C | | Spray drying at 155°C | | Spray drying at 155°C | |
| | Powder average particle diameter [µm] | 83 | | 81 | | 87 | |
| | Average particle diameter of oil agent [µm] | 0.7 | | 0.4 | | 0.4 | |
| | (Average dispersion diameter of oil agent)/(Average particle diameter of salt-sensitive particles) | 0.008 | | 0.005 | | 0.005 | |
| Evaluation | Presence or absence of oil floating | 2 | | 2 | | 2 | |
| | Moist feel | 5.0 | | 5.0 | | 5.0 | |
| | Nothing of stretched feeling | 5.0 | | 5.0 | | 5.0 | |
| | Yield of powder [%] | 42 | | 51 | | 50 | |

**Table 5**

| | | Example 16 |
|---|---|---|
| | | Dried product |
| | | mass% |
| Formulation | Acid-modified polyvinyl alcohol-1 | 6.4 |
| | Menthol | 1.1 |
| | Nonionic surfactant-1 | 0.1 |
| | Anionic surfactant | 0.1 |
| | Cellulose | 88 |
| | Ion-exchanged water | 4.3 |
| | Total | 100 |
| | Acid-modified PVA/Oil agent | 5.8 |
| Properties of dried product | Drying method | Shelf drying at 100°C |
| | Powder average particle diameter [µm] | 160 |
| | Residual rate of menthol [%] | 35 |
| | Release rate of oil agent in 10% salt water S₁₀ [%] | 34 |
| | Release rate of oil agent in 1% salt water S₁ [%] | 80 |
| | S₁ - S₁₀ [%] | 46 |
| Evaluation | Strength of feeling of coolness (menthol amount: 0.1%) | 3.25 |

The components used in Tables 1 to 5 are as follows.
- Acid-modified polyvinyl alcohol-1: GOHSENX T-330H, manufactured by The Nippon Synthetic Chemical Industry Co., Ltd., carboxylic acid-modified polyvinyl alcohol, degree of polymerization = 2,000, degree of saponification > 99 mol%
- Acid-modified polyvinyl alcohol-2: GOHSENX T-330, manufactured by The Nippon Synthetic Chemical Industry Co., Ltd., carboxylic acid-modified polyvinyl alcohol, degree of polymerization = 2,000, degree of saponification = 95 to 98 mol%
- Acid-modified polyvinyl alcohol-3: GOHSENX T-350, manufactured by The Nippon Synthetic Chemical Industry Co., Ltd., carboxylic acid-modified polyvinyl alcohol, degree of polymerization = 2,000, degree of saponification = 93 to 95 mol%
- Vinyl acetate/vinylpyrrolidone copolymer: Luviskol VA64P, manufactured by BASF SE
- Unmodified polyvinyl alcohol: PVA-117, manufactured by Kuraray Co., Ltd., degree of polymerization = 1,800, degree of saponification = 99 mol%
- Menthol: Menthol JP(TAB)COS, manufactured by Takasago International Corporation, solubility in 100 g of water = less than 0.1 g
- Nonionic surfactant-1: RHEODOL TW-S120V, manufactured by Kao Corporation, polyoxyethylene (20EO) sorbitan monostearate
- Nonionic surfactant-2: RHEODOL SP-S10V, manufactured by Kao Corporation, sorbitan stearate
- Anionic surfactant: EXTD-6NEX, manufactured by Nikko Chemicals Co., Ltd., polyoxyethylene (6EO) tridecyl ether sodium acetate
- Corn starch: Corn starch of Japanese Pharmacopoeia, JP, manufactured by Matsutani Chemical Industry Co., Ltd.
- Dipentaerythrityl trip olyhydroxystearate: SALACOS WO-6, manufactured by The Nisshin OilliO Group, Ltd., solubility in 100 g of water = less than 0.1 g
- Vaseline: Superwhite Protopet, manufactured by SONNEBORN, LLC, solubility in 100 g of water = less than 0.1 g
- Squalane: NIKKOL Squalane, manufactured by Nippon Surfactant Industries Co., Ltd., solubility in 100 g of water = less than 0.1 g
- Cellulose: KC FLOCK W-400G, manufactured by Nippon Paper Industries, Co., Ltd., average particle diameter: 24 µm
- Mica: Micromica ML-100, manufactured by Katakura & Co-op Agri Corporation
- Trehalose: Trehalose, manufactured by Hayashibara Co., Ltd.
- Dextrin: H-PDx, manufactured by Matsutani Chemical Industry Co., Ltd.

As shown in the Examples and Comparative Examples, it was confirmed that in the salt-sensitive particles of the present invention, the release rate of the oil agent in the 1% salt water is higher than the release rate of the oil agent in the 10% salt water, and when the salt concentration becomes low, the release rate of the oil agent becomes high.

In addition, it was revealed that even in the same content of the oil agent, the feeling of effects of the oil agent at the time of cleaning (during cleaning and after cleaning) is improved.

Furthermore, in the case of using menthol as the oil agent, the feeling of coolness on the cheek and forehead can be increased without increasing the burning sensation in the eyes.

Moreover, as compared with the case of direct blending, in the case of using the salt-sensitive particles, the oil floating was not found in the formulation liquid, and the storage stability was favorable because the separation of the oil agent can be suppressed.

### (Skin Cleaning Composition)

Formulation 1 and Formulation 2 used in the Examples and Comparative Examples are those shown in Table 6. Formula 1 is a formulation having the particles blended therein, and Formulation 2 is a formulation having the oil agent directly blended therein.

**Table 6**

| Component | Formulation 1 (mass%) | Formulation 2 (mass%) |
|---|---|---|
| Tromethamine | 5 | 5 |
| Arginine | 5 | 5 |
| Laureth-21 | 0.2 | 0.2 |
| Palmitic acid | 0.4 | 0.4 |
| Laureth-4 carboxylic acid | 0.11 | 0.11 |
| Propylene glycol | 5 | 5 |
| Sorbitol | 14.1 | 14.1 |
| Mannitol | 10 | 10 |
| Trehalose | 5 | 5 |
| (Acrylates/(C10-30) alkyl acrylate) crosspolymer | 0.6 | 0.6 |
| Fragrance | 0.08 | 0.08 |
| Phenoxyethanol | 0.2 | 0.2 |
| EDTA-2Na | 0.1 | 0.1 |
| Bamboo charcoal powder | 0.01 | 0.01 |
| Iron oxide | 0.1 | 0.1 |
| Salt-sensitive particles (amount of menthol of Example 1: 23.5%) | 2 | - |
| Menthol crystal | - | 0.47 |
| Water | Balance | Balance |

The skin cleaning compositions of Formulations 1 and 2 were prepared in the following manner.

Tromethamine (manufactured by ANGUS CHEMICAL COMPANY), arginine (manufactured by Ajinomoto Co., Inc.), Laureth-21 (manufactured by Kao Corporation), palmitic acid (manufactured by Kao Corporation), Laureth-4 carboxylic acid (manufactured by Kao Corporation), EDTA-2Na (manufactured by Nagase ChemteX Corporation), mannitol (manufactured by Mitsubishi Shoji Foodtech Co., Ltd.), trehalose (manufactured by Hayashibara Co., Ltd.), and water were weighed in a beaker, and the contents were heated to 50°C and completely dissolved by heating. Thereafter, the (acrylates/(C10-30) alkyl acrylate)) crosspolymer (manufactured by Lubrizol Advanced Materials, Inc.) having been dispersed in water was added to the resulting solution, followed by stirring for 30 minutes. Furthermore, the resultant was cooled to 30°C, to which were then added propylene glycol (manufactured by Adeka Corporation), phenoxyethanol (manufactured by Toho Chemical Industry Co., Ltd.), sorbitol (manufactured by Mitsubishi Shoji Foodtech Co., Ltd.), the bamboo charcoal powder (manufactured by LATEST COOPERATIVE), iron oxide (manufactured by Titan Kogyo, Ltd.), the menthol crystal (manufactured Takasago International Corporation) or the salt-sensitive particles of Example 1, and the fragrance, followed by stirring for 30 minutes. There were thus obtained skin cleaning compositions in a gel form. The pH was 10.0.

**Table 7**

| Component | Formulation 3 (mass%) | Formulation 4 (mass%) |
|---|---|---|
| Lauric acid | 1.23 | 1.23 |
| Myristic acid | 4.28 | 4.28 |
| Palmitic acid | 10.95 | 10.95 |
| Stearic acid | 8.32 | 8.32 |
| Laureth-6 carboxylic acid | 6.2 | 6.2 |
| Concentrated glycerin | 22.5 | 22.5 |
| Sorbitol | 2.4 | 2.4 |
| PEG-150 | 1 | 1 |
| Propylene glycol | 3.6 | 3.6 |
| Potassium hydroxide 48% | 8.94 | 8.94 |
| Lauryl glucoside | 2.5 | 2.5 |
| Bamboo charcoal powder | 0.01 | 0.01 |
| Salt-sensitive particles of Examples 7 to 9 (amount of menthol: 25.4% to 27.2%) | 1.7 to 1.85 | |
| Menthol crystal | | 0.47 |
| Fragrance | 0.32 | 0.32 |
| EDTA-2Na | 0.2 | 0.2 |
| Water | Balance | Balance |

The skin cleaning compositions of Formulations 3 and 4 were prepared in the following manner.

The following components: lauric acid (manufactured by Kao Corporation), myristic acid (manufactured by Kao Corporation), palmitic acid (manufactured by Kao Corporation), stearic acid (manufactured by Kao Corporation), Laureth-6 carboxylic acid (manufactured by Kao Corporation), concentrated glycerin (manufactured by Kao Corporation), sorbitol (manufactured by Mitsubishi Shoji Foodtech Co., Ltd.), PEG-150 (manufactured by NOF Corporation), and EDTA-2Na (manufactured by Nagase ChemteX Corporation) were mixed, and the contents were heated to 80°C and completely dissolved. Thereafter, the solution was neutralized with potassium hydroxide 48% (manufactured by Toagosei Co., Ltd.). Furthermore, the resultant was cooled to 30°C, to which were then added propylene glycol (manufactured by Adeka Corporation), lauryl glucoside (manufactured by Kao Corporation), the bamboo charcoal powder (manufactured by LATEST COOPERATIVE), the menthol crystal (manufactured Takasago International Corporation) or the salt-sensitive particles of Examples 7 to 9, and the fragrance, followed by stirring for 30 minutes. There were thus obtained skin cleaning compositions in a paste form. The pH was 9.6.

**Table 8**

| Component | Formulation 5 (mass%) | Formulation 6 (mass%) |
|---|---|---|
| Lauryl hydroxysultaine | 15 | 15 |
| Sorbitol | 7.5 | 7.5 |
| (Acrylates/(C10-30) alkyl acrylate) crosspolymer | 0.8 | 0.8 |
| PEG-65M | 0.02 | 0.02 |
| Potassium hydroxide 48% | 5.1 | 5.1 |
| Laureth-6 carboxylic acid | 2.4 | 2.4 |
| Lauric acid | 3.9 | 3.9 |
| Myristic acid | 1.35 | 1.35 |
| Palmitic acid | 0.3 | 0.3 |
| EDTA-2Na | 0.1 | 0.1 |
| Ethylhexyl glycerin | 1 | 1 |
| Phenoxyethanol | 0.2 | 0.2 |
| Fragrance | 0.1 | 0.1 |
| Salt-sensitive particles of Examples 7 to 9 (amount of menthol: 25.4% to 27.2%) | 1.7 to 1.85 | |
| Menthol crystal | | 0.47 |
| Water | Balance | Balance |

The skin cleaning compositions of Formulations 5 and 6 were prepared in the following manner.

The following components: lauryl hydroxysultaine (manufactured by Kao Corporation), sorbitol (manufactured by Mitsubishi Shoji Foodtech Co., Ltd.), and PEG-65M (manufactured by Meisei Chemical Works, Ltd.) were heated to 65°C and completely dissolved. The (acrylates/(C 10-30) alkyl acrylate)) crosspolymer (manufactured by Lubrizol Advanced Materials, Inc.) having been dispersed in water was added to the resulting solution, followed by stirring for 30 minutes. Thereafter, lauric acid (manufactured by Kao Corporation), myristic acid (manufactured by Kao Corporation), palmitic acid (manufactured by Kao Corporation), Laureth-6 carboxylic acid (manufactured by Kao Corporation), potassium hydroxide 48% (manufactured by Toagosei Co., Ltd,), and EDTA-2Na (manufactured by Nagase ChemteX Corporation) were added, followed by stirring for 30 minutes. Furthermore, the resultant was cooled to 30°C, to which were then added phenoxyethanol (manufactured by Toho Chemical Industry Co., Ltd.), ethylhexyl glycerin (manufactured by Kao Corporation), the menthol crystal (manufactured Takasago International Corporation) or the salt-sensitive particles of Examples 7 to 9, and the fragrance, followed by stirring for 30 minutes. There were thus obtained skin cleaning compositions in a gel form. The pH was 10.3.

### Industrial Applicability

The salt-sensitive particles of the present invention are able to improve the feeling of effects at the time of use of an oil agent to be blended in a cleaner or the like. The salt-sensitive particles of the present invention are expect to be widely applied to various products, for example, a skin cleaner, such as a facial cleaner, a body cleaner, and a solid soap; a hair cleaner, such as a shampoo; a dentifrice; a cleaner for tableware; a cleaner for clothing; a softener for clothing; and a cleaner for contact lens.

## Claims

1. Salt-sensitive particles comprising an oil agent having a solubility in 100 g of water of less than 1 g and an acid-modified polyvinyl alcohol, wherein the oil agent is dispersed in the salt-sensitive particles.

2. The salt-sensitive particles according to claim 1, wherein an average dispersion diameter of the oil agent in the salt-sensitive particles is 30 µm or less.

3. The salt-sensitive particles according to claim 1 or 2, wherein in the salt-sensitive particles, a difference (S₁ - S₁₀) between a release rate (S₁₀) of the oil agent in 10% by mass salt water and a release rate (S₁) of the oil agent in 1% by mass salt water is 12% or more.

4. The salt-sensitive particles according to any of claims 1 to 3, wherein in the salt-sensitive particles, the release rate (S₁₀) of the oil agent in 10% by mass salt water is 40% or less.

5. The salt-sensitive particles according to any of claims 1 to 4, wherein in the salt-sensitive particles, the release rate (S₁) of the oil agent in 1% by mass salt water is 30% or more.

6. The salt-sensitive particles according to any of claims 1 to 5, wherein an average particle diameter of the salt-sensitive particles is 1,500 µm or less.

7. The salt-sensitive particles according to any of claims 1 to 6, wherein a mass ratio [(acid-modified polyvinyl alcohol)/(oil agent)] of the content of the acid-modified polyvinyl alcohol to the content of the oil agent is 0.1 or more and 90 or less.

8. The salt-sensitive particles according to any of claims 1 to 7, wherein the oil is at least one selected from an alcohol, an ester oil, a hydrocarbon oil, a silicone oil, a dialkyl ether compound, an amine compound, an amide compound, oils and fats, and a higher fatty acid.

9. The salt-sensitive particles according to any of claims 1 to 8, wherein the oil agent is at least one functional oil agent selected from a refreshing agent, a moisturizing ingredient, a disinfectant, an ultraviolet absorber, and a fragrance.

10. The salt-sensitive particles according to any of claims 1 to 9, wherein a ratio of the average dispersion diameter of the oil agent to the average particle diameter of the salt-sensitive particles [(average dispersion diameter of oil agent)/(average particle diameter of salt-sensitive particles)] is 0.0005 or more and 0.3 or less.

11. A cleaner comprising the salt-sensitive particles according to any of claims 1 to 10.

12. The cleaner according to claim 11, which is used for skins.

13. A method for cleaning a skin, a hair, or a clothing, comprising using the cleaner according to claim 11.

14. A method for producing the salt-sensitive particles according to any of claims 1 to 10, comprising
a step of preparing an emulsion composition containing an oil agent having a solubility in 100 g of water of less than 1 g and an acid-modified polyvinyl alcohol; and
a step of removing the water from the emulsion composition.

15. The method for producing the salt-sensitive particles according to claim 14, comprising the following step 1-1 to step 1-3:
Step 1-1: a step of preparing an oil phase containing the oil agent;
Step 1-2: a step of preparing a water phase containing the acid-modified polyvinyl alcohol; and
Step 1-3: a step of mixing two liquids prepared in the step 1-1 and the step 1-2, to obtain the emulsion composition.
